# EUROPEAN PATENT SPECIFICATION

(11) **EP 1 720 864 B1**
(45) Date of publication and mention of the grant of the patent: **06.05.2009**
(21) Application number: 05707356.1
(22) Date of filing: 07.02.2005
(51) Int. Cl.: C07D 409/04, C07D 409/14, A61K 31/4184, A61P 29/00

(54) **BENZIMIDAZOL SUBSTITUTED THIOPHENE DERIVATIVES WITH ACTIVITY ON IKK3**
BENZIMIDAZOLSUBSTITUIERTE THIOPHENDERIVATE, DIE AUF IKK3 WIRKEN
DERIVES DE THIOPHENE A SUBSTITUTION BENZIMIDAZOLE A ACTIVITE SUR IKK3

(30) Priority: 09.02.2004 GB 0402809
(43) Date of publication of application: 15.11.2006
(73) Proprietor: GLAXO GROUP LIMITED, Greenford, Middlesex UB6 ONN (GB)
(72) Inventor: BAMBOROUGH, Paul, Stevenage Hertfordshire SG1 2NY (GB); MOREY, James, Vaughan, Stevenage Hertfordshire SG1 2NY (GB)
(74) Representative: Learoyd, Stephanie Anne
(86) International application number: PCT/EP2005/001432
(87) International publication number: WO 2005/075465

(56) References cited:
- WO-A-02/076985
- WO-A-03/029242
- WO-A-20/04014899
- DE-A1- 19 928 424

## Description

### BACKGROUND OF THE INVENTION

The present invention relates to benzimidazole derivatives, compositions and medicaments containing the same, as well as processes for the preparation and use of such compounds, compositions and medicaments. Such benzimidazole derivatives are potentially useful in the treatment of diseases associated with inappropriate I-kappa-B kinase-3 (IKK3) (also known as I-kappa-B kinase epsilon (IKKε) or inducible I-kappa B kinase (IKKi)) activity, including in particular in the treatment and prevention of disease states mediated by IKK3 mechanisms including inflammatory and tissue repair disorders, particularly rheumatoid arthritis, inflammatory bowel disease, asthma and COPD (chronic obstructive pulmonary disease); osteoarthritis, osteoporosis and fibrotic diseases; dermatosis, including psoriasis, atopic dermatitis and ultraviolet radiation (UV)-induced skin damage; autoimmune diseases including systemic lupus eythematosus, multiple sclerosis, psoriatic arthritis, alkylosing spondylitis, tissue and organ rejection, Alzheimer's disease, stroke, atherosclerosis, restonosis, diabetes, glomerulonephritis, cancer, including Hodgkins disease, cachexia, inflammation associated with infection and certain viral infections, including acquired immune deficiency syndrome (AIDS), adult respiratory distress syndrome, and Ataxia Telangiestasia.

An important large family of enzymes is the protein kinase enzyme family. Currently, there are about 500 different known protein kinases. However, because three to four percent of the human genome is a code for the formation of protein kinases, there may be many thousands of distinct and separate kinases in the human body. Protein kinases serve to catalyze the phosphorylation of an amino acid side chain in various proteins by the transfer of the γ-phosphate of the ATP-Mg²⁺ complex to said amino acid side chain. These enzymes control the majority of the signaling processes inside cells, thereby governing cell function, growth, differentiation and destruction (apoptosis) through reversible phosphorylation of the hydroxyl groups of serine, threonine and tyrosine residues in proteins. Studies have shown that protein kinases are key regulators of many cell functions, including signal transduction, transcriptional regulation, cell motility, and cell division. Several oncogenes have also been shown to encode protein kinases, suggesting that kinases play a role in oncogenesis. These processes are highly regulated, often by complex intermeshed pathways where each kinase will itself be regulated by one or more kinases. Consequently, aberrant or inappropriate protein kinase activity can contribute to the rise of disease states associated with such aberrant kinase activity. Due to their physiological relevance, variety and ubiquitousness, protein kinases have become one of the most important and widely studied family of enzymes in biochemical and medical research.

The protein kinase family of enzymes is typically classified into two main subfamilies: Protein Tyrosine Kinases and Protein Serine/Threonine Kinases, based on the amino acid residue they phosphorylate. The serine/threonine kinases (PSTK), includes cyclic AMP-and cyclic GMP-dependent protein kinases, calcium and phospholipid dependent protein kinase, calcium- and calmodulin-dependent protein kinases, casein kinases, cell division cycle protein kinases and others. These kinases are usually cytoplasmic or associated with the particulate fractions of cells, possibly by anchoring proteins. Aberrant protein serine/threonine kinase activity has been implicated or is suspected in a number of pathologies such as rheumatoid arthritis, psoriasis, septic shock, bone loss, many cancers and other proliferative diseases. Accordingly, serine/threonine kinases and the signal transduction pathways which they are part of are important targets for drug design. The tyrosine kinases phosphorylate tyrosine residues. Tyrosine kinases play an equally important role in cell regulation. These kinases include several receptors for molecules such as growth factors and hormones, including epidermal growth factor receptor, insulin receptor, platelet derived growth factor receptor and others. Studies have indicated that many tyrosine kinases are transmembrane proteins with their receptor domains located on the outside of the cell and their kinase domains on the inside. Much work is also under progress to identify modulators of tyrosine kinases as well.

The present inventors have identified novel benzimidazole derivatives which are inhibitors of kinase activity, in particular inappropriate IKK3 activity. Such derivatives are therefore of potential therapeutic benefit in the treatment of disorders associated with inappropriate kinase activity, suitably inappropriate IKK3 activity, in particular in the treatment and prevention of disease states mediated by IKK3 mechanisms including inflammatory and tissue repair disorders, particularly rheumatoid arthritis, inflammatory bowel disease, asthma and COPD (chronic obstructive pulmonary disease); osteoarthritis, osteoporosis and fibrotic diseases; dermatosis, including psoriasis, atopic dermatitis and ultraviolet radiation (UV)-induced skin damage; autoimmune diseases including systemic lupus eythematosus, multiple sclerosis, psoriatic arthritis, alkylosing spondylitis, tissue and organ rejection, Alzheimer's disease, stroke, atherosclerosis, restonosis, diabetes, glomerulonephritis, cancer, including Hodgkins disease, cachexia, inflammation associated with infection and certain viral infections, including acquired immune deficiency syndrome (AIDS), adult respiratory distress syndrome, and Ataxia Telangiestasia.

### BRIEF SUMMARY OF THE INVENTION

In one aspect of the present invention, there is provided a compound of Formula (I) selected from:

| |
|---|
| 5-[5,6-bis(methyloxy)-1H-benzimidazol-1-yl]-3-({[4-(hydroxymethyl)phenyl]methyl}oxy)-2-thiophenecarbonitrile |
| 5-[5,6-bis(methyloxy)-1*H*-benzimidazol-1-yl]-3-{[(1*R*)-1-(2-chlorophenyl)ethyl]oxy}-2-thiophenecarbonitrile |
| 5-(*1H*-benzimidazol-1-yl)-3-[(phenylmethyl)oxy]-2-thiophenecarbonitrile |
| 5-[5,6-bis(methyloxy)-*1H*-benzimidazol-1-yl]-3-([(1-methyl-*1H*-1,2,3-benzotriazol-6-yl)methyl]oxyl-2-thiophenecarbonitrile) |
| 5-(6-(methyloxy)-5-{[2-(4-morpholinyl)ethyl]oxy}-*1H*-benzimidazol-1-yl)-3-({[2-(trifluoromethyl)phenyl]methyl}oxy)-2-thiophenecarbonitrile |
| 5-[5,6-bis(methyloxy)-*1H*-benzimidazol-1-yl]-3-{[(2,5-difluorophenyl)methyl]oxy}-2-thiophenecarbonitrile |
| 5-[5,6-bis(methyloxy)-*1H*-benzimidazol-1-yl]-3-({[2-(methylsulfonyl)phenyl]methyl}oxy)-2-thiophenecarbonitrile |
| 5-(5-chloro-*1H*-benzimidazol-1-yl)-3-[(phenylmethyl)oxy]-2-thiophenecarbonitrile |
| 5-[5,6-bis(methyloxy)-*1H*-benzimidazol-1-yl]-3-{[(2,6-difluorophenyl)methyl]oxy}-2-thiophenecarbonitrile |
| 5-[5-[(3-hydroxypropyl)oxy]-6-(methyloxy)-*1H*-benzimidazol-1-yl]-3-({[2-(trifluoromethyl)phenyl]methyl}oxy)-2-thiophene-carbonitrile |
| 5-[5,6-bis(methyloxy)-*1H*-benzimidazol-1-yl]-3-{[(3-bromo-4-pyridinyl)methyl]oxy}-2-thiophenecarbonitrile |
| 5-[5,6-bis(methyloxy)-*1H*-benzimidazol-1-yl]-3-[(cyclohexylmethyl)oxy]-2-thiophenecarbonitrile |
| 1-[5-cyano-4-({[2-(trifluoromethyl)phenyl]methyl}oxy)-2-thienyl]-N-[2-(4-morpholinyl)ethyl]-*1H*-benzimidazole-5-carboxamide |
| 5-[5,6-bis(methyloxy)-*1H*-benzimidazol-1-yl]-3-({(1*R*)-1-[2-(trifluoromethyl)phenyl]ethyl}oxy)-2-thiophenecarbonitrile |
| 5-[5,6-bis(methyloxy)-*1H*-benzimidazol-1-yl]-3-[(2-phenylethyl)oxy]-2-thiophenecarbonitrile |
| 5-[5,6-bis(methyloxy)-*1H*-benzimidazol-1-yl]-3-[({2-[(trifluoromethyl)oxy]phenyl}methyl)oxy]-2-thiophenecarbonitrile |
| 5-[5,6-bis(methyloxy)-*1H*-benzimidazol-1-yl]-3-({[2-(methyloxy)phenyl]methyl}oxy)-2-thiophenecarbonitrile |
| 5-[5,6-bis(methyloxy)-*1H*-benzimidazol-1-yl]-3-{[2-(4-morpholinyl)ethyl]oxy}-2-thiophenecarbonitrile |
| 5-[5,6-bis(methyloxy)-*1H*-benzimidazol-1-yl]-3-{[2-(2-oxo-1-pyrrolidinyl)ethyl]oxy}-2-thiophenecarbonitrile |
| 5-[5,6-bis(methyloxy)-*1H*-benzimidazol-1-yl]-3-[(tetrahyd ro-2-furanylmethyl)oxy]-2-thiophenecarbonitrile |
| 5-[5,6-bis(methyloxy)-*1H*-benzimidazol-1-yl]-3-[(tetrahydro-*2H*-pyran-2-ylmethyl)oxy]-2-thiophenecarbonitrile |
| 5-[5,6-bis(methyloxy)-*1H*-benzimidazol-1-yl]-3-{[2-(phenyloxy)ethyl]oxy}-2-thiophenecarbonitrile |
| 5-[5,6-bis(methyloxy)-*1H*-benzimidazol-1-yl]-3-{[(1*S*)-1-(2-chlorophenyl)butyl]oxy}-2-thiophenecarbonitrile |
| 5-[5,6-bis(methyloxy)-*1H*-benzimidazol-1-yl]-3-[(3-thienylmethyl)oxy]-2-thiophenecarbonitrile |
| 5-[5,6-bis(methyloxy)-*1H*-benzimidazol-1-yl]-3-[(2-thienylmethyl)oxy]-2-thiophenecarbonitrile |
| 5-[5,6-bis(methyloxy)-*1H*-benzimidazol-1-yl]-3-{[(1*R*)-1-methylpropyl]oxy}-2-thiophenecarbonitrile |

and pharmaceutically acceptable salts or solvates thereof.

In a second aspect of the present invention, there is provided a compound of Formula (I), or a salt or solvate thereof for use in therapy.

In a third aspect of the present invention, there is provided a pharmaceutical composition comprising a therapeutically effective amount of a compound of Formula (I) or a salt or solvate thereof and one or more of pharmaceutically acceptable carriers, diluents and excipients.

The compounds of the invention fall in the scope of reference of Formula (Ia) 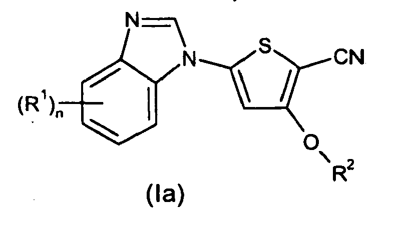 wherein
n is 0, 1, 2, 3, or 4;
Each R¹ which may be the same or different, independently represents H, halogen or a group (X)ₐ(Y)_{b}Z;
X represents -0- or -CONH-;
a is 0 or1;
Y represents -C₁₋₆ alkylene-
b is 0 or 1;
Z represents hydroxy, C₁₋₆ alkyl, C₁₋₆ haloalkyl, C₅₋₇ heterocyclyl, C₁₋₆ alkoxyalkyl, C₁₋₆ haloalkoxyalkyl;
R² represents a group -(X¹)c(Y¹)_{d}Z¹;
Wherein X¹ represents -C₁₋₆ alkylene-;
c is 0 or1;
Y¹ represents -O-;
d is 0 or1;
Z¹ represents H, aryl or heteroaryl each of which contains 5 - 14 ring members, C₅₋₇ heterocyclyl, C₅₋₇ cycloalkyl, C₅₋₇ cycloalkenyl (each of which aryl, heteroaryl, heterocyclyl, cycloalkyl, cycloalkeny may be optionally substituted by one or more substituents independently selected from C₁₋₆ alkyl, C₁₋₆ haloalkyl, halogen, C₁₋₆ alkoxy, C₁₋₆ haloalkoxy, SO₂R³, C₁₋₆ hydroxyalkyl)
R³ represents H or C₁₋₆ alkyl;
or pharmaceutically acceptable salts and solvates thereof.

In a further aspect there is provided the use of a compound of Formula (I) or a salt or solvate thereof in the manufacture of a medicament for the treatment of inflammatory and tissue repair disorders, particularly rheumatoid arthritis, inflammatory bowel disease, asthma and COPD (chronic obstructive pulmonary disease); osteoarthritis, osteoporosis and fibrotic diseases; dermatosis, including psoriasis, atopic dermatitis and ultraviolet radiation (UV)-induced skin damage; autoimmune diseases including systemic lupus eythematosus, multiple sclerosis, psoriatic arthritis, alkylosing spondylitis, tissue and organ rejection, Alzheimer's disease, stroke, atherosclerosis, restonosis, diabetes, glomerulonephritis, cancer, including Hodgkins disease, cachexia, inflammation associated with infection and certain viral infections, including acquired immune deficiency syndrome (AIDS), adult respiratory distress syndrome, and Ataxia Telangiestasia.

In a further aspect there is provided a compound of Formula (1) or a salt or solvate thereof for use in treating a disorder mediated by inappropriate IKK3 activity, suitably treatment of inflammatory and tissue repair disorders, particularly rheumatoid arthritis, inflammatory bowel disease, asthma and COPD (chronic obstructive pulmonary disease); osteoarthritis, osteoporosis and fibrotic diseases; dermatosis, including psoriasis, atopic dermatitis and ultraviolet radiation (UV)-induced skin damage; autoimmune diseases including systemic lupus eythematosus, multiple sclerosis, psoriatic arthritis, alkylosing spondylitis, tissue and organ rejection, Alzheimer's disease, stroke, atherosclerosis, restonosis, diabetes, glomerulonephritis, cancer, including Hodgkins disease, cachexia, inflammation associated with infection and certain viral infections, including acquired immune deficiency syndrome (AIDS), adult respiratory distress syndrome, and Ataxia Telangiestasia.

### DETAILED DESCRIPTION OF THE INVENTION

As used herein, the term "effective amount" means that amount of a drug or pharmaceutical agent that will elicit the biological or medical response of a tissue, system, animal or human that is being sought, for instance, by a researcher or clinician. Furthermore, the term "therapeutically effective amount" means any amount which, as compared to a corresponding subject who has not received such amount, results in improved treatment, healing, prevention, or amelioration of a disease, disorder, or side effect, or a decrease in the rate of advancement of a disease or disorder. The term also includes within its scope amounts effective to enhance normal physiological function.

As used herein "a compound of the invention" or "a compound of Formula (I) means a compound of Formula (I) or a pharmaceutically acceptable salt or solvate thereof.

As used herein the term "alkyl" refers to a straight- or branched-chain hydrocarbon radical having the specified number of carbon atoms, so for example, as used herein, the terms "C₁-C₃ alkyl" and "C₁-C₆ alkyl" refer to an alkyl group, as defined above, containing at least 1, and at most 3 or 6 carbon atoms respectively. Examples of such branched or straight-chained alkyl groups useful in the present invention include, but are not limited to, methyl, ethyl, n-propyl, isopropyl, isobutyl, n-butyl, t-butyl, n-pentyl, isopentyl, and n-hexyl.

As used herein, the term "alkylene" refers to a straight or branched chain divalent hydrocarbon radical having the specified number of carbon atoms, so for example, as used herein, the terms "C₁-C₃ alkylene" and "C₁-C₆ alkylene" refer to an alkylene group, as defined above, which contains at least 1, and at most 3 or 6, carbon atoms respectively. Examples of "C₁-C₃ alkylene" and "C₁-C₆ alkylene" groups useful in the present invention include, but are not limited to, methylene, ethylene, n-propylene, n-butylene, isopentylene, and the like.

As used herein, the term "alkenyl" (and "alkenylene") refers to straight or branched hydrocarbon chains containing the specified number of carbon atoms and at least one and up to 3 carbon-carbon double bonds. Examples include ethenyl (and ethenylene) and propenyl (and propenylene).

As used herein, the term "halogen" refers to fluorine (F), chlorine (Cl), bromine (Br), or iodine (I) and the term "halo" refers to the halogen radicals: fluoro (-F), chloro (-Cl), bromo(-Br), and iodo(-I).

As used herein, the term "C₁-C₆ haloalkyl" refers to a straight or branched chain alkyl group as defined above containing at least 1, and at most 6 carbon atoms respectively substituted with at least one halo group, halo being as defined herein. Examples of such branched or straight chained haloalkyl groups useful in the present invention include, but are not limited to, methyl, ethyl, propyl, isopropyl, isobutyl and n-butyl substituted independently with one or more halos, e.g., fluoro, chloro, bromo and iodo.

As used herein, the term "cycloalkyl" refers to a non-aromatic cyclic hydrocarbon ring containing the specified number of carbon atoms so, for example, the term "C₅-C₇ cycloalkyl" refers to a non-aromatic cyclic hydrocarbon ring having from five to seven carbon atoms. Exemplary "C₅-C₇ cycloalkyl" groups useful in the present invention include, but are not limited to, cyclopentyl, cyclohexyl and cycloheptyl.

As used herein, the term "cycloalkenyl" refers to a non-aromatic monocyclic carboxycyclic ring having the specified number of carbon atoms and up to 3 carbon-carbon double bonds. "Cycloalkenyl" includes by way of example cyclopentenyl and cyclohexenyl.

As used herein, the term "heterocyclic" or the term "heterocyclyl" refers to a non-aromatic heterocyclic ring containing the specified number ring atoms being saturated or having one or more degrees of unsaturation, containing one or more heteroatom substitutions selected from O and/or N. Such a ring may be optionally fused to one or more other "heterocyclic" ring(s) or cycloalkyl ring(s). Examples of "heterocyclic" moieties include, but are not limited to, tetrahydrofuran, pyran, 1,4-dioxane, 1,3-dioxane, piperidine, piperazine, 2,4-piperazinedione, pyrrolidine, imidazolidine, pyrazolidine, morpholine, thiomorpholine, tetrahydrothiopyran, tetrahydrothiophene, and the like.

As used herein, the term "aryl" refers to monocyclic carbocyclic groups and fused bicyclic carbocyclic groups having the specified number of carbon atoms and having at least one aromatic ring. Examples of aryl groups include phenyl and naphthyl.

As used herein, the term "heteroaryl" refers to an aromatic monocyclic ring, or to a fused bicyclic or tricyclic ring system wherein at least one ring is aromatic, having the specified number of ring atoms and containing at least one heteratom selected from N, O, and/or S. Examples of "heteroaryl" groups used herein include furanyl, thiophenyl, pyrrolyl, imidazolyl, pyrazolyl, triazolyl, tetrazolyl, thiazolyl, oxazolyl, isoxazolyl, oxadiazolyl, oxo-pyridyl, thiadiazolyl, isothiazolyl, pyridyl, pyridazyl, pyrazinyl, pyrimidyl, quinolinyl, isoquinolinyl, benzofuranyl, benzothiophenyl, indolyl, indazolyl.

As used herein, the term "alkoxy" refers to the group RₐO-, where Rₐ is alkyl as defined above and the terms "C₁-C₃ alkoxy" and "C₁-C₆ alkoxy" refer to an alkoxy group as defined herein wherein the alkyl moiety contains at least 1, and at most 3 or 6, carbon atoms. Exemplary "C₁-C₃ alkoxy" and "C₁-C₆ alkoxy" groups useful in the present invention include, but are not limited to, methoxy, ethoxy, n-propoxy, isopropoxy, n-butoxy, and t-butoxy.

As used herein the term "hydroxyalkyl" refers to the group -RₐOH, where Rₐ is an alkylene group as defined above.

As used herein, the term "haloalkoxy" refers to the group RₐO-, where Rₐ is haloalkyl as defined above and the term "C₁-C₆ haloalkoxy" refers to a haloalkoxy group as defined herein wherein the haloalkyl moiety contains at least 1, and at most 6, carbon atoms. Exemplary C₁-C₆ haloalkoxy groups useful in the present invention include, but is not limited to, trifluoromethoxy.

As used herein, the term "optionally" means that the subsequently described event(s) may or may not occur, and includes both event(s), which occur, and events that do not occur.

As used herein, the term "solvate" refers to a complex of variable stoichiometry formed by a solute (in this invention, a compound of Formula (I) or a salt or physiologically functional derivative thereof) and a solvent. Such solvents for the purpose of the invention may not interfere with the biological activity of the solute. Examples of suitable solvents include, but are not limited to, water, methanol, ethanol and acetic acid. Preferably the solvent used is a pharmaceutically acceptable solvent. Examples of suitable pharmaceutically acceptable solvents include, without limitation, water, ethanol and acetic acid. Most preferably the solvent used is water.

As used herein, the term "substituted" refers to substitution with the named substituent or substituents, multiple degrees of substitution being allowed unless otherwise stated.

Certain of the compounds described herein may contain one or more chiral atoms, or may otherwise be capable of existing as two enantiomers. The compounds of this invention include mixtures of enantiomers as well as purified enantiomers or enantiomerically enriched mixtures. Also included within the scope of the invention are the individual isomers of the compounds represented by Formula (I) above as well as any wholly or partially equilibrated mixtures thereof. The present invention also covers the individual isomers of the compounds represented by the formulas above as mixtures with isomers thereof in which one or more chiral centers are inverted. Also, it is understood that any tautomers and mixtures of tautomers of the compounds of Formula (I) are included within the scope of the compounds of Formula (I).

The present invention also covers salts of the compounds of Formula (I). Typically, the salts of the present invention are pharmaceutically acceptable salts. Salts encompassed within the term "pharmaceutically acceptable salts" refer to non-toxic salts of the compounds of this invention. Salts of the compounds of the present invention may comprise acid addition salts derived from a nitrogen on a substituent in the compound of Formula (I). Representative salts include the following salts: acetate, benzenesulfonate, benzoate, bicarbonate, bisulfate, bitartrate, borate, bromide, calcium edetate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isethionate, lactate, lactobionate, laurate, malate, maleate, mandelate, mesylate, methylbromide, methylnitrate, methylsulfate, monopotassium maleate, mucate, napsylate, nitrate, N-methylglucamine, oxalate, palmoate (embonate), palmitate, pantothenate, phosphate/diphosphate, polygalacturonate, potassium, salicylate, sodium, stearate, subacetate, succinate, tannate, tartrate, teoclate, tosylate, triethiodide, trimethylammonium and valerate. Other salts, which are not pharmaceutically acceptable, may be useful in the preparation of compounds of this invention and these form a further aspect of the invention.

While it is possible that, for use in therapy, therapeutically effective amounts of a compound of Formula (I), as well as salts and solvates thereof, may be administered as the raw chemical, it is possible to present the active ingredient as a pharmaceutical composition. Accordingly, the invention further provides pharmaceutical compositions, which include therapeutically effective amounts of compounds of the Formula (I) and salts and solvates thereof, and one or more pharmaceutically acceptable carriers, diluents, or excipients. The compounds of the Formula (I) and salts and solvates thereof, are as described above. The carrier(s), diluent(s) or excipient(s) must be acceptable in the sense of being compatible with the other ingredients of the formulation and not deleterious to the recipient thereof. In accordance with another aspect of the invention there is also provided a process for the preparation of a pharmaceutical composition including admixing a compound of the Formula (I), or salts and solvates thereof, with one or more pharmaceutically acceptable carriers, diluents or excipients.

Pharmaceutical compositions may be presented in unit dose forms containing a predetermined amount of active ingredient per unit dose. Such a unit may contain, for example, 0.5mg to 1g, preferably 1mg to 700mg, more preferably 5mg to 100mg of a compound of the Formula (I), depending on the condition being treated, the route of administration and the age, weight and condition of the patient, or pharmaceutical compositions may be presented in unit dose forms containing a predetermined amount of active ingredient per unit dose. Preferred unit dosage compositions are those containing a daily dose or sub-dose, as herein above recited, or an appropriate fraction thereof, of an active ingredient. Furthermore, such pharmaceutical compositions may be prepared by any of the methods well known in the pharmacy art.

Pharmaceutical compositions may be adapted for administration by any appropriate route, for example by the oral (including buccal or sublingual), rectal, nasal, topical (including buccal, sublingual or transdermal), vaginal or parenteral (including subcutaneous, intramuscular, intravenous or intradermal) route. Such compositions may be prepared by any method known in the art of pharmacy, for example by bringing into association the active ingredient with the carrier(s) or excipient(s).

Pharmaceutical compositions adapted for oral administration may be presented as discrete units such as capsules or tablets; powders or granules; solutions or suspensions in aqueous or non-aqueous liquids; edible foams or whips; or oil-in-water liquid emulsions or water-in-oil liquid emulsions.

For instance, for oral administration in the form of a tablet or capsule, the active drug component can be combined with an oral, non-toxic pharmaceutically acceptable inert carrier such as ethanol, glycerol, water and the like. Powders are prepared by comminuting the compound to a suitable fine size and mixing with a similarly comminuted pharmaceutical carrier such as an edible carbohydrate, as, for example, starch or mannitol. Flavoring, preservative, dispersing and coloring agent can also be present.

Capsules are made by preparing a powder mixture, as described above, and filling formed gelatin sheaths. Glidants and lubricants such as colloidal silica, talc, magnesium stearate, calcium stearate or solid polyethylene glycol can be added to the powder mixture before the filling operation. A disintegrating or solubilizing agent such as agar-agar, calcium carbonate or sodium carbonate can also be added to improve the availability of the medicament when the capsule is ingested.

Moreover, when desired or necessary, suitable binders, lubricants, disintegrating agents and coloring agents can also be incorporated into the mixture. Suitable binders include starch, gelatin, natural sugars such as glucose or beta-lactose, corn sweeteners, natural and synthetic gums such as acacia, tragacanth or sodium alginate, carboxymethylcellulose, polyethylene glycol, waxes and the like. Lubricants used in these dosage forms include sodium oleate, sodium stearate, magnesium stearate, sodium benzoate, sodium acetate, sodium chloride and the like. Disintegrators include, without limitation, starch, methyl cellulose, agar, bentonite, xanthan gum and the like. Tablets are formulated, for example, by preparing a powder mixture, granulating or slugging, adding a lubricant and disintegrant and pressing into tablets. A powder mixture is prepared by mixing the compound, suitably comminuted, with a diluent or base as described above, and optionally, with a binder such as carboxymethylcellulose, an aliginate, gelatin, or polyvinyl pyrrolidone, a solution retardant such as paraffin, a resorption accelerator such as a quaternary salt and/or an absorption agent such as bentonite, kaolin or dicalcium phosphate. The powder mixture can be granulated by wetting with a binder such as syrup, starch paste, acadia mucilage or solutions of cellulosic or polymeric materials and forcing through a screen. As an alternative to granulating, the powder mixture can be run through the tablet machine and the result is imperfectly formed slugs broken into granules. The granules can be lubricated to prevent sticking to the tablet forming dies by means of the addition of stearic acid, a stearate salt, talc or mineral oil. The lubricated mixture is then compressed into tablets. The compounds of the present invention can also be combined with a free flowing inert carrier and compressed into tablets directly without going through the granulating or slugging steps. A clear or opaque protective coating consisting of a sealing coat of shellac, a coating of sugar or polymeric material and a polish coating of wax can be provided. Dyestuffs can be added to these coatings to distinguish different unit dosages.

Oral fluids such as solution, syrups and elixirs can be prepared in dosage unit form so that a given quantity contains a predetermined amount of the compound. Syrups can be prepared by dissolving the compound in a suitably flavored aqueous solution, while elixirs are prepared through the use of a non-toxic alcoholic vehicle. Suspensions can be formulated by dispersing the compound in a non-toxic vehicle. Solubilizers and emulsifiers such as ethoxylated isostearyl alcohols and polyoxy ethylene sorbitol ethers, preservatives, flavor additive such as peppermint oil or natural sweeteners or saccharin or other artificial sweeteners, and the like can also be added.

Where appropriate, dosage unit compositions for oral administration can be microencapsulated. The composition can also be prepared to prolong or sustain the release as for example by coating or embedding particulate material in polymers, wax or the like.

The compounds of Formula (I) and salts and solvates thereof, can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

The compounds of Formula (I) and salts and solvates thereof may also be delivered by the use of monoclonal antibodies as individual carriers to which the compound molecules arse coupled. The compounds may also be coupled with soluble polymers as targetable drug carriers. Such polymers can include polyvinylpyrrolidone, pyran copolymer, polyhydroxypropylmethacrylamide -phenol, polyhydroxyethylaspartamidephenol, or polyethyleneoxidepolylysine substituted with palmitoyl residues. Furthermore, the compounds may be coupled, to a class of biodegradable polymers useful in achieving controlled release of a drug, for example, polylactic acid, polepsilon caprolactone, polyhydroxy butyric acid, polyorthoesters, polyacetals, polydihydropyrans, polycyanoacrylates and cross-linked or amphipathic block copolymers of hydrogels.

Pharmaceutical compositions adapted for transdermal administration may be presented as discrete patches intended to remain in intimate contact with the epidermis of the recipient for a prolonged period of time. For example, the active ingredient may be delivered from the patch by iontophoresis as generally described in Pharmaceutical Research, 3(6), 318 (1986).

Pharmaceutical compositions adapted for topical administration may be formulated as ointments, creams, suspensions, lotions, powders, solutions, pastes, gels, sprays, aerosols or oils.

For treatments of the eye or other external tissues, for example mouth and skin, the compositions are preferably applied as a topical ointment or cream. When formulated in an ointment, the active ingredient may be employed with either a paraffinic or a water-miscible ointment base. Alternatively, the active ingredient may be formulated in a cream with an oil-in-water cream base or a water-in-oil base.

Pharmaceutical compositions adapted for topical administrations to the eye include eye drops wherein the active ingredient is dissolved or suspended in a suitable carrier, especially an aqueous solvent.

Pharmaceutical compositions adapted for topical administration in the mouth include lozenges, pastilles and mouth washes.

Pharmaceutical compositions adapted for rectal administration may be presented as suppositories or as enemas.

Pharmaceutical compositions adapted for nasal administration wherein the carrier is a solid include a coarse powder having a particle size for example in the range 20 to 500 microns which is administered in the manner in which snuff is taken, i.e. by rapid inhalation through the nasal passage from a container of the powder held close up to the nose. Suitable compositions wherein the carrier is a liquid, for administration as a nasal spray or as nasal drops, include aqueous or oil solutions of the active ingredient.

Pharmaceutical compositions adapted for administration by inhalation include fine particle dusts or mists, which may be generated by means of various types of metered, dose pressurised aerosols, nebulizers or insufflators.

For administration by inhalation the compounds according to the invention are conveniently delivered in the form of spray compositions. Spray compositions may for example be formulated as aqueous solutions or suspensions, for example for nebulisation, or as aerosols delivered from pressurised packs, such as a metered dose inhaler, with the use of a suitable liquefied propellant. Aerosol compositions suitable for inhalation can be either a suspension or a solution and generally contain a compound of the invention and a suitable propellant such as a fluorocarbon or hydrogen-containing chlorofluorocarbon or mixtures thereof, particularly hydrofluoroalkanes, especially 1,1,1,2-tetrafluoroethan, 1,1,1,2,3,3,3-heptafluoro-n-propan or a mixture thereof. The aerosol composition may optionally contain additional formulation excipients well known in the art such as surfactants e.g. oleic acid or lecithin and cosolvents eg. Ethanol.

Advantageously, the compositions of the invention may be buffered by the addition of suitable buffering agents.

Capsules and cartridges for use in an inhaler or insufflator, of for example gelatine, may be formulated containing a powder mix for inhalation of a compound of the invention and a suitable powder base such as lactose or starch. Alternatively, the compound of the invention may be presented without excipients such as lactose.

Aerosol formulations are preferably arranged so that each metered dose or "puff' of aerosol contains a particular amount of a compound of the invention. Administration may be once daily or several times daily, for example 2, 3 4 or 8 times, giving for example 1, 2 or 3 doses each time. The overall daily dose and the metered dose delivered by capsules and cartridges in an inhaler or insufflator will generally be double those with aerosol formulations.

Pharmaceutical compositions adapted for vaginal administration may be presented as pessaries, tampons, creams, gels, pastes, foams or spray formulations.

Pharmaceutical compositions adapted for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The compositions may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets.

It should be understood that in addition to the ingredients particularly mentioned above, the compositions may include other agents conventional in the art having regard to the type of composition in question, for example those suitable for oral administration may include flavouring agents.

A therapeutically effective amount of a compound of the present invention will depend upon a number of factors including, for example, the age and weight of the animal, the precise condition requiring treatment and its severity, the nature of the formulation, and the route of administration, and will ultimately be at the discretion of the attendant physician or veterinarian. However, an effective amount of a compound of Formula (I) or (Ia) will generally be in the range of 0.1 to 100 mg/kg body weight of recipient (mammal) per day and more usually in the range of 1 to 10 mg/kg body weight per day. Thus, for a 70kg adult mammal, the actual amount per day would usually be from 70 to 700 mg and this amount may be given in a single dose per day or more usually in a number (such as two, three, four, five or six) of sub-doses per day such that the total daily dose is the same. An effective amount of a salt or solvate, or physiologically functional derivative thereof, may be determined as a proportion of the effective amount of the compound of Formula (I) per se.

The compounds of Formula (I) and salts and solvates thereof, are believed to have utility in as a result of inhibition of the protein kinase IKK3, in particular in the treatment and prevention of disease states mediated by IKK3 mechanisms including inflammatory and tissue repair disorders, particularly rheumatoid arthritis, inflammatory bowel disease, asthma and COPD (chronic obstructive pulmonary disease); osteoarthritis, osteoporosis and fibrotic diseases; dermatosis, including psoriasis, atopic dermatitis and ultraviolet radiation (UV)-induced skin damage; autoimmune diseases including systemic lupus eythematosus, multiple sclerosis, psoriatic arthritis, alkylosing spondylitis, tissue and organ rejection, Alzheimer's disease, stroke, atherosclerosis, restonosis, diabetes, glomerulonephritis, cancer, including Hodgkins disease, cachexia, inflammation associated with infection and certain viral infections, including acquired immune deficiency syndrome (AIDS), adult respiratory distress syndrome, and Ataxia Telangiestasia.

The present invention thus also provides compounds of Formula (I) and pharmaceutically acceptable salts or solvates thereof, for use in medical therapy, and particularly in the treatment of disorders mediated by IKK3 activity.

The inappropriate IKK3 activity referred to herein is any IKK3 activity that deviates from the normal IKK3 activity expected in a particular mammalian subject. Inappropriate IKK3 activity may take the form of, for instance, an abnormal increase in activity, or an aberration in the timing and or control of IKK3 activity. Such inappropriate activity may result then, for example, from overexpression or mutation of the protein kinase leading to inappropriate or uncontrolled activation.

The present invention is directed to methods of regulating, modulating, or inhibiting IKK3 for the prevention and/or treatment of disorders related to unregulated IKK3 activity. In particular, the compounds of the present invention can also be used in inflammatory disease, particularly rheumatoid arthritis.

A further aspect of the invention provides a method of treatment of a mammal suffering from a disorder mediated by IKK3 activity, which includes administering to said subject an effective amount of a compound of Formula (I) or a pharmaceutically acceptable salt or solvate thereof.

A further aspect of the present invention provides the use of a compound of Formula (I), or a pharmaceutically acceptable salt or solvate thereof, in the preparation of a medicament for the treatment of a disorder characterized by inappropriate IKK3 activity.

Particular disorders characterised by inappropriate IKK3 activity in particular in the treatment and prevention of disease states mediated by IKK3 mechanisms including inflammatory and tissue repair disorders, particularly rheumatoid arthritis, inflammatory bowel disease, asthma and COPD (chronic obstructive pulmonary disease); osteoarthritis, osteoporosis and fibrotic diseases; dermatosis, including psoriasis, atopic dermatitis and ultraviolet radiation (UV)-induced skin damage; autoimmune diseases including systemic lupus eythematosus, multiple sclerosis, psoriatic arthritis, alkylosing spondylitis, tissue and organ rejection, Alzheimer's disease, stroke, atherosclerosis, restonosis, diabetes, glomerulonephritis, cancer, including Hodgkins disease, cachexia, inflammation associated with infection and certain viral infections, including acquired immune deficiency syndrome (AIDS), adult respiratory distress syndrome, and Ataxia Telangiestasia. as a result of inhibition of the protein kinase IKK3.

The compounds of this invention may be made by a variety of methods, including standard chemistry. Any previously defined variable will continue to have the previously defined meaning unless otherwise indicated. Illustrative general synthetic methods are set out below and then specific compounds of the invention are prepared in the Working Examples.

Compounds of general Formula (I) and reference Formula (Ia) may be prepared by methods known in the art of organic synthesis as set forth in part by the following synthesis schemes. In all of the schemes described below, it is well understood that protecting groups for sensitive or reactive groups are employed where necessary in accordance with general principles of chemistry. Protecting groups are manipulated according to standard methods of organic synthesis (T. W. Green and P. G. M. Wuts (1991) Protecting Groups in Organic Synthesis, John Wiley & Sons). These groups are removed at a convenient stage of the compound synthesis using methods that are readily apparent to those skilled in the art. The selection of processes as well as the reaction conditions and order of their execution shall be consistent with the preparation of compounds of Formula (I) and (Ia). Those skilled in the art will recognize if a stereocenter exists in compounds of Formula (I) and (Ia). Accordingly, the present invention includes both possible stereoisomers and includes not only racemic compounds but the individual enantiomers as well. When a compound is desired as a single enantiomer, it may be obtained by stereospecific synthesis or by resolution of the final product or any convenient intermediate. Resolution of the final product, an intermediate, or a starting material may be effected by any suitable method known in the art. See, for example, Stereochemistry of Organic Compounds by E. L. Eliel, S. H. Wilen, and L. N. Mander (Wiley-Interscience, 1994).

Compounds of Formula I and reference formula (Ia) can be prepared for example according to the general scheme depicted below and the Examples section following:

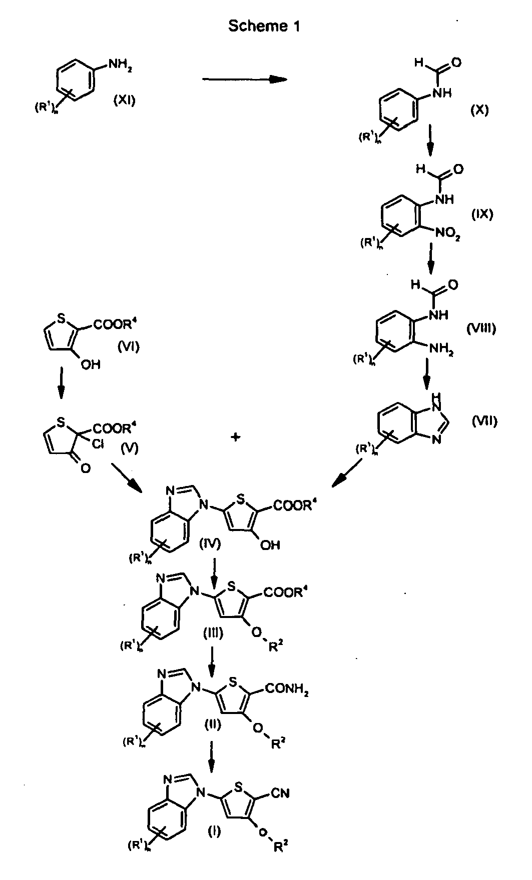

A compound of Formula (1) or reference formula (Ia) may be prepared by dehydration of a Compound of Formula (II) 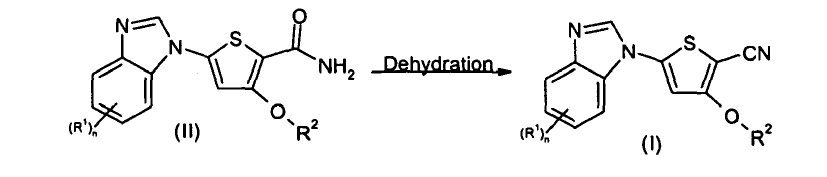 wherein all variables are as defined for a compound of Formula (I) and (Ia).

The dehydration reaction can be carried out using a variety of reagents. Suitable dehydration reagents include but are not limited to thionyl chloride, trifluoroacetic anhydride, phosphorous oxychloride, phosphorous pentoxide, and *N,N-*dicyclohexylcarbodiimide and 2-chloro-1,3-dimethylimidazolinium chloride. The reaction may be optionally heated to from about 50 to about 150 °C. Suitable solvents for this reaction include but are not limited to dichloromethane, chloroform, benzene, toluene, N,N-dimethylformamide and dichloroethane.

A compound of Formula (II) may be prepared directly from a compound of Formula (III). 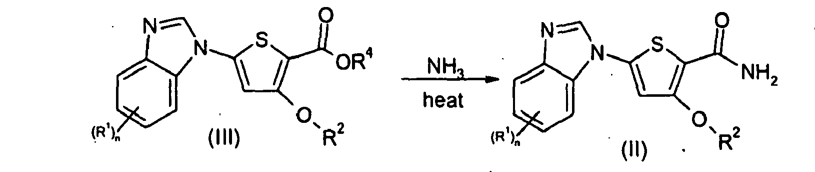 wherein all variables are as defined in connection with Formula (1) and (Ia). R⁴ represents for example, a suitable carboxylic acid protecting group, C₁₋₆ alkyl, C₁₋₆ alkenyl, C₁₋₆ alkynyl or C₅₋₇ cycloalkyl.

This reaction is typically performed in a sealed vessel with an excess of ammonia. The reaction is typically heated to a temperature of from about 50 to about 120 °C. Suitable solvents for this reaction include but are not limited to methanol, ethanol, isopropanol, tetrahydrofuran, and dioxane.

A compound of Formula (III) may be prepared from compounds of Formula (IV): 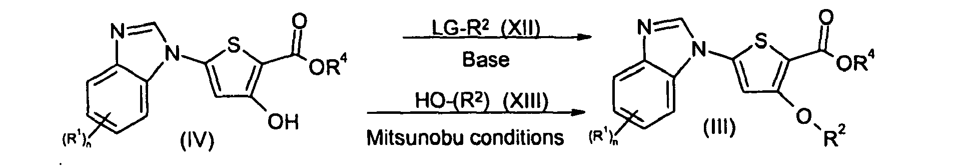 LG is a suitable leaving group; and
all other variables are as defined in connection with Scheme 1 above.

In general the process for preparing a compound of Formula (III) comprises the steps of:
a) reacting the compound of Formula (IV) with a base and a compound of Formula (XII) to prepare a compound of the Formula (III); or
b) reacting the compound of Formula (IV) with a compound of Formula (XIII) under Mitsunobu conditions to prepare a compound of Formula (III).

More specifically, a compound of Formula (III) can be prepared by reacting a compound of Formula (IV) with a compound of Formula (XII). The compounds of Formula (XII) are commercially available or can be prepared using conventional knowledge in the art. The reaction may be carried out in an inert solvent, conveniently at room temperature, in the presence of a suitable base. The compound of Formula (IV) and the compound of Formula (XII) may be present in equimolar amounts; however, a slight excess of the compound of Formula (XII) may be employed if desired. Examples of suitable bases for this reaction include but are not limited to, potassium carbonate, sodium carbonate, cesium carbonate, sodium hydride, and potassium hydride. Examples of suitable inert solvents for this reaction include but are not limited to, *N,N*-dimethylformamide, tetrahydrofuran, dioxane, and 1,2-dimethoxyethane.

In another embodiment, a compound of Formula (III) can be prepared by reacting a compound of Formula (IV) with a compound of Formula (XIII). The compounds of Formula (XIII) are commercially available or can be prepared using conventional knowledge in the art. The reaction is carried out in an inert solvent under standard Mitsunobu conditions. *See*, Hughes, D.L., Org. React. 42:335-656 (1992); and Mitsunobu, O., Synthesis 1-28 (1981). Typically the compound of Formula (III), the compound of Formula (XII), a triarylphosphine, and a dialkyl azodicarboxylate are reacted together at room temperature. Examples of suitable triarylphosphines include but are not limited to, triphenylphosphine, tri-*p*-tolylphosphine, and trimesitylphosphine. Examples of suitable dialkyl azodicarboxylates include but are not limited to, diethyl azodicarboxylate, diisopropyl azodicarboxylate, and di-*tert*-butyl azodicarboxylate. Examples of suitable inert solvents for this reaction include but are not limited to, tetrahydrofuran, dioxane, 1,2-dimethoxyethane, dichloromethane, and toluene.

More specifically, compounds of Formula (IV) can be prepared by reacting a compound of Formula (VII) with a compound of Formula (V) to prepare a compound of Formula (IV). 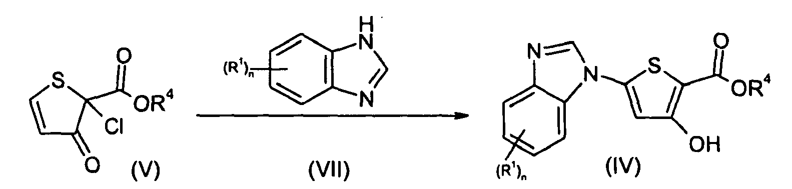 wherein all variables are as defined as previously.

The reaction of a compound of Formula (VII) with a compound of Formula (V) is typically carried out in an inert solvent at room temperature. Typically two molar equivalents of a compound of Formula (VII) are combined with one molar equivalent of a compound of Formula (V). Examples of suitable inert solvents for this reaction include but are not limited to, chloroform, dichloromethane, tetrahydrofuran, dioxane, and toluene.

A compound of Formula (V) can be prepared by reacting a compound of Formula (VI) with sulfuryl chloride. 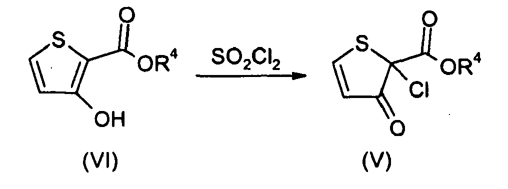 wherein all variables are as defined as previously.

Compounds of Formula (VI) are commercially available or can be prepared using conventional knowledge in the art. Typically, reaction of a compound of Formula (VI) with sulfuryl chloride at room temperature provides a compound of Formula (V). Excess sulfuryl chloride may be used if desired. Examples of suitable solvents include but are not limited to chloroform, dichloromethane, and toluene. *See*, Corral, C.; Lissavetzky, J. Synthesis 847-850 (1984).

A compound of Formula VII may be prepared according to the process outlined in the Scheme below: 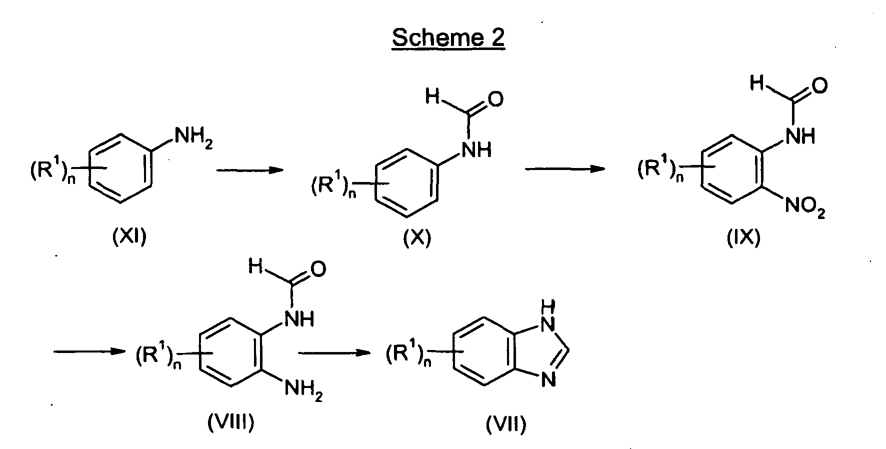 wherein all variables are as defined as previously.

Generally, this process for preparing the a compound of Formula (VII) (all formulas and all variables having been defined above comprises the steps of:
a) reacting a compound of Formula (XI) with a suitable acylating agent to prepare a compound of the Formula (X);
b) reacting a compound of Formula (X) under nitration conditions to prepare a compound of the Formula (IX);
c) reducing a compound of Formula (IX) to prepare a compound of Formula (VIII); and
d) cyclizing a compound of Formula (VIII) to prepare a compound of Formula (VII).

The order of the foregoing steps is not critical to the practice of the invention and the process may be practiced by performing the steps in any suitable order based on the knowledge of those skilled in the art.

More specifically, a compound of Formula (VII) can be prepared by cyclizing a compound of Formula (VIII): 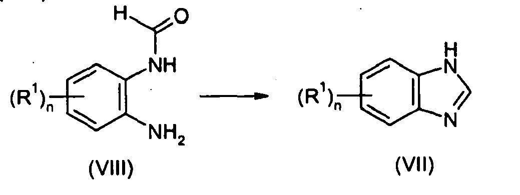 wherein all variables are as defined above.

This type of cyclization reaction is well documented in the literature. See, Braña, M. F., et. al., J. Med. Chem. 45: 5813-5816 (2002); Fonseca, T., et. al., Tetrahedron 57: 1793-1799 (2001); White, A. W., et. al., J. Med. Chem. 43: 4084-4097 (2000); and Tamura, S. Y., et. al., Biorg. Med. Chem. Lett. 7: 1359-1364 (1997). This reaction may be carried out neat or in a suitable solvent. The reaction may optionally be heated to a temperature of from about 50 to about 200 °C. Typically an excess of a suitable acid is used. Examples of suitable acids include but are not limited to acetic acid, trifluoroacetic acid, hydrochloric acid, hydrobromic acid, sulfuric acid, methanesulfonic acid, p-toluenesulfonic acid, and pyridinium p-toluenesulfonate. A dehydrating reagent may optionally be used as well. Examples of suitable dehydrating reagents include but are not limited to magnesium sulfate, sodium sulfate, phosphorous pentoxide, and molecular sieves. Examples of suitable solvents include but are not limited to dichloromethane, chloroform, toluene, xylenes, methanol, ethanol, and water.

A compound of Formula (VIII) may be prepared by reducing a compound of Formula (IX). 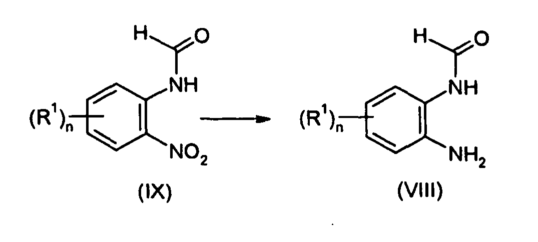 wherein all variables are as defined above.

The reduction can be carried out using conventional techniques and reducing agents. See, Rangarajan, M., et al., Bioorg, Med. Chem. 8:2591-2600 (2000); White, A.W., et al., J. Med. Chem. 43: 4084-4097 (2000); Silvestri, R., et al., Bioorg. Med. Chem. 8:2305-2309 (2000); Nagaraja, D., et al., Tetrahedron Lett. 40:7855-7856 (1999); Jung, F., et al., J. Med. Chem. 34:1110-1116 (1991); Srivastava, R.P., et al., Pharmazie 45:34-37 (1990); Hankovszky, H.O., et al., Can. J. Chem. 67:1392-1400 (1989); Ladd, D.L., et al., J. Org. Chem. 53:417-420 (1988); Mertens, A., et al., J. Med. Chem. 30:1279-1287 (1987); and Sharma, K.S., et al., Synthesis 4:316-318 (1981). Examples of suitable reducing agents for this reaction include but are not limited to, palladium with hydrogen, palladium with ammonium formate, platinum oxide with hydrogen, nickel with hydrogen, tin(II) chloride, iron with acetic acid, aluminum with ammonium chloride, borane, sodium dithionite, and hydrazine. The reaction may optionally be heated to between about 50 and about 120 °C. Suitable solvents for this reaction vary and include but are not limited to, water, methanol, ethanol, ethyl acetate, tetrahydrofuran, and dioxane.

A compound of Formula (IX) may be prepared by reacting a compound of Formula (X) under nitration conditions. 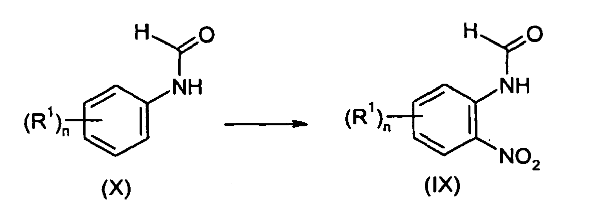 wherein all variables are as defined above.

The compound of Formula (IX) can be prepared by reacting a compound of Formula (X) under nitration conditions.

The protection of anilines e.g. in Formula (X) by the moiety -COR⁵ (wherein R⁵ is a protecting group not H as depicted above) is a common transformation well known to one skilled in the art. *See*, Kocienski, P.J. Protecting Groups, Georg Thieme Verlag, Stuttgart, 1994; and Greene, T.W., Wuts, P. G. M. Protecting Groups in Organic Synthesis (2nd Edition), J. Wiley and Sons, 1991. Suitable protecting groups for this application include but are not limited to acetyl, trifluoroacetyl, benzyloxycarbonyl, allyloxycarbonyl, 2-(rimethylsilyl)ethoxycarbonyl, phenylsulfonyl, and p-toluenesulfonyl. Reagents and conditions vary according to the nature of the particular protecting group. Some typical reagents include but are not limited to acetic anhydride, trifluoroacetic anhydride, benzyl chloroformate, allyl chloroformate, 4-nitrophenyl 2-(trimethylsilyl)ethyl carbonate, phenylsulfonyl chloride, and p-toluensulfonyl chloride. In certain cases the addition of some base is required. Examples of suitable bases include but are not limited to potassium carbonate, sodium carbonate, trialkylamines, pyridine, and potassium t-butoxide. Suitable solvents for these conversions include but are not limited to dichloromethane, chloroform, tetrahydrofuran, acetic acid, methanol, ethanol, water, toluene, and diethyl ether.

The nitration of anilines is also well documented in the literature and the foregoing reaction may be carried out using these conventional techniques. See, Wissner, A., et. al., J. Med. Chem. 46: 49-63 (2003); Duggan, S. A., et. al., J. Org. Chem. 66: 4419-4426 (2001); Clews, J., et. al., Tetrahedron 56: 8735-8746 (2000); and Kagechika, H., J. Med. Chem. 31: 2182-2192 (1988). The nitration may be carried out with a variety of nitrating reagents including but not limited to 70% aqueous nitric acid, red fuming nitric acid, ammonium nitrate with trifluoroacetic anhydride, and potassium nitrate with trifluoromethanesulfonic acid. The reaction is typically conducted at room temperature, but may be optionally heated to a temperature of from about 40 to about 100 °C in certain cases. Suitable solvents include but are not limited to acetic acid, sulfuric acid, acetic anhydride, dichloromethane, and chloroform.

The nitration results in a compound of Formula (IX), Formula (VII) can be accomplished through many different conventional methods. See, Kocienski, P.J. Protecting Groups, Georg Thieme Verlag, Stuttgart, 1994; and Greene, T.W., Wuts, P. G. M. Protecting Groups in Organic Synthesis (2nd Edition), J. Wiley and Sons, 1991.

A compound of Formula (X) may be prepared by acylating a compound of Formula (XI).
wherein all variables are as defined above. 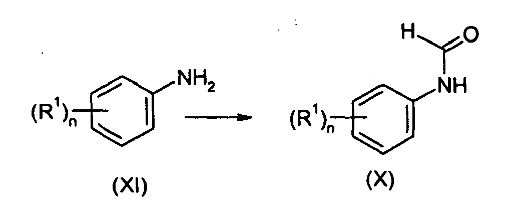 Acylation of anilines is a common transformation well known to one skilled in the art and such conventional acylation techniques may be employed for carrying out the foregoing reaction. See, Larock, R. C. Comprehensive Organic Transformations, VCH Publishers, Inc., New York, pp. 972-976, 979, 981 (1989). The acylation reaction is typically carried out using an acylating agent such as an acid halide, acid anhydride, or carboxylic acid, in the presence of a coupling reagent(s). Examples of suitable coupling reagents include but are not limited to *N,N'*-dicyclohexylcarbodiimide, 1-(3-dimethylaminopropyl)-3-ethylcarbodiimide hydrochloride, *O*-(7-azabenzotriazol-1-yl)-*N,N,N';N'*-tetramethyluronium hexafluorophosphate, and *N,N'*-carbonyldiimidazole. Suitable solvents include but are not limited to *N,N* dimethylformamide, tetrahydrofuran, dioxane, toluene, benzene, 1,2-dimethoxyethane, and 1-methyl-2-pyrrolidinone. Anilines of Formula (XI) are commercially available or readily prepared from commercially available material using conventional techniques.

Reaction conditions will be apparent to a skilled person and Examples of suitable conditions are further exemplified in the Examples Section below. It will also be evident from the above scheme that a compound of Formula (I) may be converted into another compound of Formula (I) using conventional procedures.

Certain embodiments of the present invention will now be illustrated by way of example only. The physical data given for the compounds exemplified is consistent with the assigned structure of those compounds.

### EXAMPLES

As used herein the symbols and conventions used in these processes, schemes and examples are consistent with those used in the contemporary scientific literature, for example, the *Journal of the American Chemical Society* or the *Journal of Biological Chemistry.* Standard single-letter or three-letter abbreviations are generally used to designate amino acid residues, which are assumed to be in the L-configuration unless otherwise noted. Unless otherwise noted, all starting materials were obtained from commercial suppliers and used without further purification. Specifically, the following abbreviations may be used in the examples and throughout the specification:

| | |
|---|---|
| g (grams); | mg (milligrams); |
| L (liters); | mL (milliliters); |
| µL (microliters); | psi (pounds per square inch); |
| M (molar); | mM (millimolar); |
| i. v. (intravenous); | Hz (Hertz); |
| MHz (megahertz); | mol (moles); |
| mmol (millimoles); | rt (room temperature); |
| min (minutes); | h (hours); |
| mp (melting point); | TLC (thin layer chromatography); |
| Tᵣ (retention time); | RP (reverse phase); |
| MeOH (methanol); | *i-*PrOH (isopropanol); |
| TEA (triethylamine); | TFA (trifluoroacetic acid); |
| TFAA (trifluoroacetic anhydride); | THF (tetrahydrofuran); |
| DMSO (dimethylsulfoxide); | AcOEt (ethyl acetate); |
| DME (1,2-dimethoxyethane); | DCM (dichloromethane); |
| DCE (dichloroethane); | DMF (N,N-dimethylformamide); |
| DMPU (*N,N'*-dimethylpropyleneurea); | CDI (1,1-carbonyldiimidazole); |
| IBCF (isobutyl chloroformate); | HOAc (acetic acid); |
| HOSu (*N*-hydroxysuccinimide); | HOBT (1-hydroxybenzotriazole); |
| mCPBA (meta-chloroperbenzoic acid; | |
| EDC (1-[3-dimethylamino) propyl]-3-ethylcarbodiimide hydrochloride); | |
| BOC (*tert*-butyloxycarbonyl); FMOC (9-fluorenylmethoxycarbonyl); | |
| DCC (dicyclohexylcarbodiimide); | CBZ (benzyloxycarbonyl); |
| Ac (acetyl); | atm (atmosphere); |
| TMSE (2-(trimethylsilyl)ethyl); | TMS (trimethylsilyl); |
| TIPS (triisopropylsilyl); | TBS (*t*-butyldimethylsilyl); |
| DMAP (4-dimethylaminopyridine); | BSA (bovine serum albumin) |
| ATP (adenosine triphosphate); | HRP (horseradish peroxidase); |
| DMEM (Dulbecco's modified Eagle medium); | |
| HPLC (high pressure liquid chromatography); | |
| BOP (bis(2-oxo-3-oxazolidinyl)phosphinic chloride); | |
| TBAF (tetra-n-butylammonium fluoride); | |
| HBTU(O-Benzotriazole-1-yl-N,N,N',N'-tetramethyluroniumhexafluoro phosphate). | |
| HEPES (4-(2-hydroxyethyl)-1-piperazine ethane sulfonic acid); | |
| DPPA (diphenylphosphoryl azide); | |
| fHNO₃ (fuming HNO₃); and | |
| EDTA (ethylenediaminetetraacetic acid). | |
| TMEDA (N,N,N',N'-tetramethyl-1,2-ethanediamine) | |
| NBS (N-bromosuccinimide) | |
| HATU (O-(7azabenzobenzotriazol-1-yl)-N,N,N',N'-tetramethyluronium | |
| hexafluorophosphate) | |
| DIPEA (diisopropylethylamine) | |
| dppf (1,1'-bis(diphenylphosphino)ferrocene) | |
| NIS (N-iodsuccinimide) | |
| PTFE ((poly)tetrafluoroethylene) | |
| LC-MS (liquid chromatography - mass spectrometry) | |

All references to ether are to diethyl ether; brine refers to a saturated aqueous solution of NaCl. Unless otherwise indicated, all temperatures are expressed in °C (degrees Centigrade). All reactions are conducted under an inert atmosphere at room temperature unless otherwise noted.

¹H NMR spectra were recorded on a Bruker DPX400 spectrometer using the specified solvent. Chemical shifts are expressed in parts per million (ppm, δ units). Splitting patterns describe apparent multiplicities and are designated as s (singlet), d (doublet), t (triplet), quartet (q), quint. (quintet), sext. (sextet) m (multiplet), br (broad), c (complex).

Low-resolution mass spectra (MS) were recorded on a JEOL JMS-AX505HA, JEOL SX-102, or a SCIEX-APliii spectrometer; LC-MS were recorded on Waters ZQ spectrometers.

Preparative HPLC refers to methods where the material was purified by High Performance Liquid Chromatography on a HPLC ABZ+ 5µm column (10 cm x 21.2 mm i.d.) with 0.1% formic acid in water and 0.05% formic acid in acetonitrile utilising gradient elution at a flow rate of 8 mL/min and UV detection at 254 nm.

Mass directed auto preparation chromatography refers to methods where the material was purified by HPLC comprised of the following apparatus: Waters 600 gradient pump, Waters 2700 sample manager, Waters reagent manager, Micromass ZQ mass spectrometer, Gilson 202 - fraction collector, Gilson 202 - waste collector, Gilson 115 post-fraction UV detector. Detection is by UV and fraction collection is triggered by observation of the programmed mass ion for the compound of interest. Software used is Micromass Masslynx version 3.5. Two columns are used: For polar (< 1.8 min analytical RT) compounds, an Optimal ODS-L is used, whose dimensions are 20mm internal diameter by 100mm in length. The stationary phase particle size is 5 µm. For all other compounds, a Supelco LCABZ++ column is employed, whose dimensions are 20mm internal diameter by 100 mm in length. The stationary phase particle size is 5µm. Eluting solvents are: water + 0.1% formic acid and acetonitrile: water 95:5+0.05% formic acid; using a runtime of 14 minutes and a flow rate of 20 mL/min.

### Intermediate 1: Methyl 2-chloro-3-oxo-2,3-dihydro-2-thiophenecarboxylate

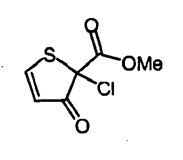

To a solution of methyl 3-hydroxy-2-thiophenecarboxylate (5.00 g, 31.6 mmol) in chloroform (10 mL) was added 1M sulfuryl chloride in dichloromethane (34.8 mL, 34.8 mmol) dropwise over 2 minutes under a N₂ atmosphere. The mixture was stirred for 4 hours at room temperature and the volatiles removed under reduced presssure. The solids were recrystallized from hexane to give methyl 2-chloro-3-oxo-2,3-dihydro-2-thiophenecarboxylate (4.60 g, 76%) as white needles. ¹H NMR (CDCl₃): δ 8.38 (d, 1 H), 6.23 (d, 1 H), 3.84 (s, 3 H); MS *m*/*z* 193 (M+1).

### Example

**(1) 5-[5,6-Bis(methyloxy)-1*H*-benzimidazol-1-yl]-3-({[4-(hydroxymethyl)phenyl]methyl}oxy)-2-thiophenecarbonitrile** 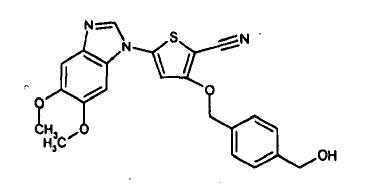 To a solution of 5-[5,6-bis(methyloxy)-1*H*-benzimidazol-1-yl]-3-({[4-(hydroxymethyl)-phenyl]methyl}oxy)-2-thiophenecarboxamide (22.0 mg, 0.05 mmol) and pyridine (121 µL, 1.50 mmol) in dichloromethane (5 mL) cooled to approximately -10°C (ice/sodium chloride/water bath) was added trifluoroacetic anhydride (70 µL, 0.5 mmol) and the mixture stirred for 5 mins. after which the solution was allowed to warm up to room temperature over 16 h. The reaction mixture was quenched with saturated aqueous sodium hydrogen carbonate solution (1 mL) and shaken. The organic layer was recovered by passing the mixture through a PTFE phase separator, collected in a tared glass vial and concentrated to dryness by nitrogen blow down at 40 °C. The resulting crude solid was dissolved in 1:1 dimethyl sulfoxide/methanol solution (500 µL) and purified by mass directed auto preparation chromatography to afford the title compound as an off white solid (6.9 mg). MS [M+1]⁺ 422.29; ¹H NMR δ_{H} (400.13 MHz, *d*₆-DMSO, TMS): 8.47 (s, 1 H), 7.73 (s, 1 H), 7.47 (d, *J* = 8.0 Hz, 2 H), 7.38 (d, *J* = 8.0 Hz, 2 H), 7.35 (s, 1 H), 7.23 (s, 1 H), 5.41 (s, 2 H), 5.22 (t, *J* = 5.5 Hz, 1 H), 4.52 (d, *J* = 5.5 Hz, 2 H), 3.85 (s, 3 H), 3.83 (s, 3 H).
**(2) 5-[5,6-Bis(methyloxy)-1*H-*benzimidazol-1-yl]-3-{[(1*R*)-1-(2-chlorophenyl)ethyloxy}-2-thiophenecarbonitrile** 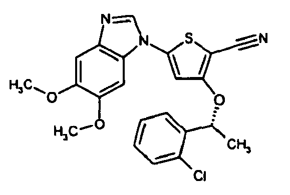 To a solution of 5-[5,6-bis(methyloxy)-1*H*-benzimidazol-1-yl]-3-{[(1*R*)-1-(2-chlorophenyl)-ethyl]oxy}-2-thiophenecarboxamide (31.1 mg, 0.07 mmol) and pyridine (156 µL, 1.93 mmol) in dichloromethane (5 mL) cooled to approximately -10°C (ice/sodium chloride/water bath) was added trifluoroacetic anhydride (408 µL, 2.89 mmol) and the mixture stirred for 1 hr. after which the solution was poured into ice/water (10 mL). After all the ice had melted, potassium carbonate (500 mg) was added and the solution extracted with neat dichloromethane (10 mL). The organic phase was recovered by passing through a PTFE phase separator and taken down to dryness by nitrogen blow down at 40 °C. The crude solid was dissolved in 1:1 dimethyl sulfoxide/methanol solution (2 mL) and purified by preparative HPLC to afford the title compound as a white solid (7.0 mg). MS [M+1]⁺ 440.07 + 442.03 (characteristic Cl isotope pattern); ¹H NMR δ_{H} (400.13 MHz, CD₃CN, TMS): 7.96 (s, 1 H), 7.63 (dd, *J* = 1.3, 7.8 Hz, 1 H), 7.44-7.31 (c m, 3 H), 7.26 (s, 1 H), 7.01 (s, 1 H), 6.95 (s, 1 H), (5.99, q, *J* = 6.5 Hz, 1 H), 3.86 (s, 3 H), 3.82 (s, 3 H), 1.71 (d, *J* = 6.3Hz, 3H)
**(3) 5-(1*H*-Benzimidazol-1-yl)-3-{[(2-methylphenyl)methyl]oxy}-2-thiophenecarbonitrile (Reference Example)** 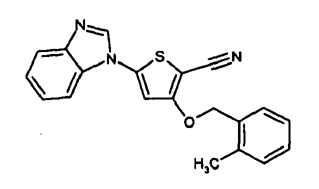 5-(1*H*-Benzimidazol-1-yl)-3-[(2-methylbenzyl)oxy]-2-thiophenecarboxamide (28.5 mg, 0.08 mmol) was dissolved in pyridine (2 mL) and cooled to 0 °C. Trifluoroacetic anhydride (17 µL, 0.120 mmol) was added dropwise via syringe. The mixture was stirred for 15 min. and warmed to room temperature. After 1 h. dichloromethane (2 mL) was added followed by five drops of trifluoroacetic anhydride were added to dissolve the insoluble components of the mixture. After 14 h. the reaction was poured into dichloromethane and brine. The layers were separated and the aqueous layer was washed twice with dichloromethane. The combined organic layers were dried over magnesium sulphate, filtered, and concentrated *in vacuo.* Purification by flash chromatography gave the title compound as a pale yellow solid (7.5 mg) MS [M+1]⁺ 346; ¹H NMR δ_{H} (400.13 MHz, *d*₆-DMSO, TMS): 8.71 (s, 1H). 7.83 (s and m, 3H), 7.49-7.25 (m, 6H), 5.44 (s, 2H), 2.40 (s, 3H).
**(4) 5-(1*H*-Benzimidazol-1-yl)-3-[(phenylmethyl)oxy]-2-thiophenecarbonitrile** 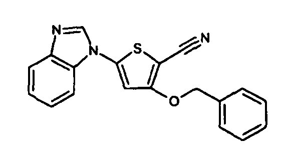 To a solution of 5-(1*H*-Benzimidazol-1-yl)-3-[(phenylmethyl)oxy]-2-thiophenecarboxamide (250 mg, 0.72 mmol) in dichloromethane (5 mL) was added 2-chloro-1,3-dimethylimidazolinium chloride (125 mg, 0.74 mmol) and trifluoroacetic acid (55 µL, 0.74 mmol). To this solution was added triethylamine (200 µL, 1.43 mmol). The mixture was stirred for 3 h. at room temperature, then for 3 h. at reflux. The reaction mixture was cooled to room temperature and additional 2-chloro-1,3-dimethylimidazolinium chloride (125 mg, 0.74 mmol) and trifluoroacetic acid (55 µL, 0.74 mmol) were added, followed by triethylamine (200 µL, 1.43 mmol). The mixture was stirred for 14 h. then poured into water and extracted with dichloromethane. The organic extracts were washed with 5% aqueous hydrochloric acid, then saturated aqueous sodium hydrogen carbonate, then saturated aqueous sodium chloride solution and dried over sodium sulphate. Filtration and concentration *in vacuo,* followed by silica gel chromatography (eluting with a 20 to 50 % ethyl acetate/hexane gradient) afforded the title compound as a white solid (186 mg). MS [M+1]⁺ 332; ¹H NMR δ_{H} (400.13 MHz, *d*₆-DMSO, TMS): 8.69 (s, 1H), 7.79-7.82 (m, 2H), 7.76 (s, 1H), 7.49-7.53 (m, 2H), 7.37-7.47 (m, 5H), 5.42 (s, 2H).
**(5) 5-[5,6-Bis(methyloxy)-1*H*-benzimidazol-1-yl]-3-({[2-(trifluoromethyl)phenyl]methyl}oxy)-2-thiophenecarbonitrile** 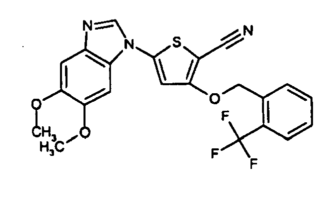 To a solution of 5-(5,6-dimethoxy-1*H*-benzimidazol-1-yl)-3-{[2-(trifluoromethyl)-benzyl]oxy}thiophene-2-carboxamide (150 mg, 0.31 mmol) in dichloromethane (2 mL) was added 2-chloro-1,3-dimethylimidazolinium chloride (120 mg, 0.71 mmol) and trifluoroacetic acid (50 µL, 0.65 mmol). To this solution was added triethylamine (200 µL, 1.44 mmol). The mixture was stirred for 18 h. then additional 2-chloro-1,3-dimethylimidazolinium chloride (120 mg, 0.71 mmol) and trifluoroacetic acid (50 µL, 0.65 mmol) were added, followed by triethylamine (200 µL, 1.44 mmol). The mixture was stirred for 4 h then poured into water and extracted with dichloromethane. The organic extracts were washed with 5% aqueous hydrochloric acid, then saturated aqueous sodium hydrogen carbonate, then saturated aqueous sodium chloride solution and dried over sodium sulphate. Filtration and concentration followed by silica gel chromatography (eluting with a 40 to 95% ethyl acetate/hexane gradient) afforded the title compound as a yellow solid (66 mg). MS [M+1]⁺ 459; ¹H NMR δ_{H} (400.13 MHz, CDCl₃, TMS): δ 7.90 (s, 1 H), 7.80-7.72 (m, 2 H), 7.66 (t, *J* = 7.6 Hz, 1 H), 7.51 (t, *J* = 7.5 Hz, 1 H), 7.31 (s, 1 H), 6.99 (s, 1 H), 6.82 (s, 1 H), 5.55 (s, 2 H), 3.96 (s, 3 H), 3.92 (s, 3 H).
**(6) 5-[5,6-bis(methyloxy)-1*H*-benzimidazol-1-yl]-3-{[(1-methyl-1*H*-1,2,3-benzotriazol-6-yl)methyl]oxy}-2-thiophenecarbonitrile** 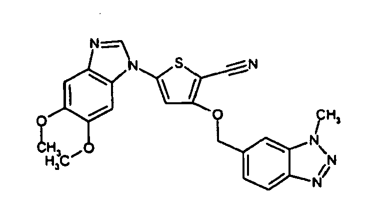 Prepared in a manner analogous to procedure *(1)* using 5-[5,6-bis(methyloxy)-1*H-*benzimidazol-1-yl]-3-{[(1-methyl-1*H*-1,2,3-benzotriazol-6-yl)methyl]oxy}-2-thiophene-carboxamide (23.3 mg, 0.05 mmol). The resulting crude solid was dissolved in 1:1 dimethyl sulfoxide/methanol solution (500 µL) and purified by mass directed auto preparation chromatography to afford the title compound as a white solid (6.2 mg). MS [M+1]⁺447.28; ¹H NMR δ_{H} (400.13 MHz, *d*₆-DMSO, TMS): 8.48 (s, 1 H), 8.11 (d, *J* = 8.5 Hz, 1 H), 8.01 (s, 1 H), 7.77 (s, 1 H), 7.55 (dd, *J* = 8.5, 1.3 Hz, 1 H), 7.35 (s, 1 H), 7.24 (s, 1 H), 5.61 (s, 2 H), 4.33 (s, 3 H), 3.83 (s, 3 H), 3.81 (s, 3 H).
**(7) 5-(6-(Methyloxy)-5-{[2-(4-morpholinyl)ethyl]oxy}-1*H*-benzimidazol-1-yl)-3-({[2-(trifluoromethyl)pheny]methyl}oxy)-2-thiophene carbonitrile** 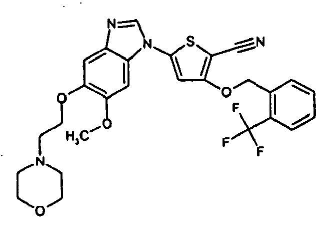 Prepared in an a manner analogous to procedure *(1)* using 5-(6-(methyloxy)-5-{[2-(4-morpholinyl)ethyl]oxy}-1*H* benzimidazol-1-yl)-3-({[2-(trifluoromethyl)phenyl]methyl}oxy)-2-thiophenecarboxamide (17.5 mg, 0.03 mmol) The crude solid was triturated from 1:1 dimethyl sulfoxide/methanol (500 µL), collected by suction Filtration on a PTFE phase separator, washed well with methanol and dried under suction in air to afford the title compound as an off-white solid (12.2 mg). MS [M+1]⁺ 559.37; ¹H NMR δ_{H} (400.13 MHz, *d*₆-DMSO, TMS): 8.55 (s, 1 H), 7.87-7.78 (c m, 4H), 7.67 (m, 1 H), 7.55 (s, 1 H), 7.35 (s, 1 H), 5.56 (s, 2 H), 4.39 (br. s, 2 H), 4.01 (br. s, 2 H), 3.89 (s, 3 H), 3.82-3.17 (br. c m, 8H, overlapping with residual H₂O
***(8)* 5-[5,6-Bis(methylooxy)-1*H*-benzimidazol-1-yl]-3-{[2,5 difluorophenyl)methyl]oxy}-2-thiophenecarbonitrile (Reference Example)** 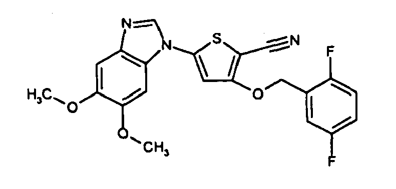 Prepared in manner analogous to procedure *(1)* using 5-[5,6-bis(methyloxy)-1*H-*benzimidazol-1-yl]-3-{[(2,5-difluorophenyl)methyl]-oxy}-2-thiophenecarboxamide (22.3 mg, 0.05 mmol) and the title compound afforded by the trituration procedure described in *(7)* as an off-white solid (9.2 mg). MS [M+1]⁺ 428.24; ¹H NMR δ_{H} (400.13 MHz, *d*₆-DMSO, TMS): 8.48 (s, 1 H), 7.79 (s, 1 H), 7.50 (c m, 1 H) 7.42-7.32, (c m, 3 H), 7.28 (s, 1 H), 5.47 (s, 2 H), 3.86 (s, 3 H), 3.83 (s, 3 H).
**(9) 5-[5,6-Bis(methyloxy)-1*H*-benzimidazol-1-yl]-3-({[2-(methylsulfonyl)phenyl]methyl}oxy)-2-thiophenecarbonitrile** 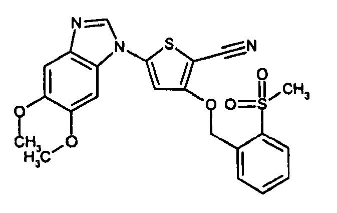 Prepared in a manner analogous to procedure *(1)* using 5-[5,6-bis(methyloxy)-1*H-*benzimidazol-1-yl]-3-({[2-(methylsulfonyl)phenyl]-methyl}oxy)-2-thiophenecarboxamide (24.5 mg, 0.05 mmol) The resulting crude solid was dissolved in 1:1 dimethyl sulfoxide/methanol solution (500 µL) and purified by mass directed auto preparation chromatography to afford the title compound as an off-white solid (4.6 mg). MS [M+1]⁺ 470.25; ¹H NMR δ_{H} (400.13 MHz, *d*₆-DMSO, TMS): 8.50 (s, 1 H), 8.06 (d, *J* = 7.8, 1 H), 7.86-7.83 (c m, 2 H), 7.76-7.72 (cm, 2 H), 7.36 (s, 1 H), 7.28 (s, 1 H), 5.82 (s, 2 H), 3.86 (s, 3 H), 3.83 (s, 3 H), 3.35 (s, 3 H)
**(10) 5-(5-Chloro-1*H*-benzimidazol-1-yl)-3-[(phenylmethyl)oxy]-2-thiophenecarbonitrile** 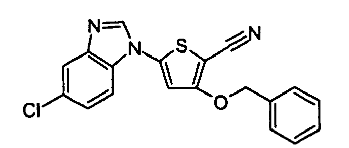 Prepared in a manner analogous to procedure *(4)* using 5-(5-Chloro-1*H*-benzimidazol-1-yl)-3-[(phenylmethyl)oxy]-2-thiophenecarboxamide (105 mg, 0.270 mmol). Silica gel chromatography (eluting with a 10 to 40% solution of ethyl acetate in hexane) afforded the title compound as an off-white solid (71 mg) MS [M+1]⁺ 366; ¹H NMR δ_{H} (400.13MHz, *d*₆-DMSO, TMS): 8.75 (s, 1 H), 7.91 (d, *J* = 1.8 Hz, 1 H), 7.79-7.82 (m, 1 H), 7.70 (s, 1 H), 7.39-7.52 (m, 6 H), 5.41 (s, 2 H).
**(11) 5-[5,6-bis(Methyloxy)-1*H*-benzimidazol-1-yl]-3-{[(2,6-difluorophenyl)methyl]oxy}-2-thiophenecarbonitrile** 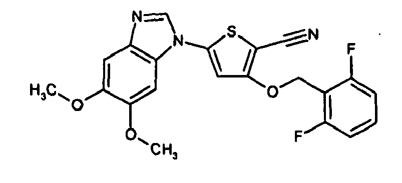 Prepared in a manner analogous to procedure *(1)* using 5-[5,6-bis(methyloxy)-1*H-*benzimidazol-1-yl]-3-{[(2,6-difluorophenyl)-methyl]-oxy}-2-thiophenecarboxamide (22.4 mg, 0.05 mmol) and the title compound afforded by the trituration procedure described in *(7)* as an off-white solid (9.9 mg). MS [M+1]⁺428.24; ¹H NMR δ_{H} (400.13 MHz, d₆-DMSO, TMS): 8.48 (s, 1 H), 7.81 (s, 1 H), 7.60 (tt, *J* = 8.5, 6.8 Hz, 1 H), 7.36 (s, 1 H), 7.33 (s, 1 H), 7.25 (t, *J* = 8.0 Hz, 2 H), 5.49 (s, 2 H), 3.88 (s, 3 H), 3.83 (s, 3 H).
**(12) 5-[5-[(3-Hydroxypropyl)oxy]-6-(methyloxy)-1*H*-benzimidazol-1-yl]-3-({[2-(trifluoromethyl)phenyl]methyl}oxy)-2-thiophenecarbonitrile** 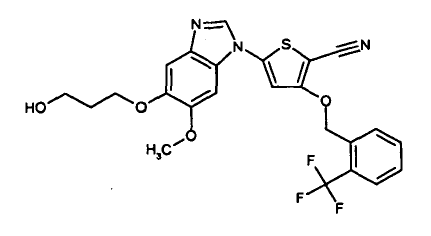 Prepared in a manner analogous to procedure *(1)* using 5-[5-[(3-hydroxypropyl)oxy]-6-(methyloxy)-1*H*-benzimidazol-1-yl]-3-({[2-(trifluoromethyl)phenyl]methyl}oxy)-2-thiophenecarboxamide (26.1 mg, 0.05 mmol) The resulting crude solid was dissolved in 1:1 dimethyl sulfoxide/methanol solution (500 µL) and purified by mass directed auto preparation chromatography to afford the title compound as an off-white solid (5.4 mg). MS [M+1]⁺ 504.27; ¹H NMR δ_{H} (400.13 MHz, *d*₆-DMSO, TMS): 8.49 (s, 1 H), 7.86-7.78 (c m, 4H), 7.66 (m, 1 H), 7.35 (s, 1 H), 7.28 (s, 1 H), 5.56 (s, 2 H), 4.55 (t, *J* = 5.0 Hz, 1 H), 4.09 (t, *J* = 6.3 Hz, 2 H), 3.86 (s, 3 H), 3.58 (dd, *J* = 6.0, 5.5 Hz, 2 H), 1.90 (quint., *J* = 6.3 Hz, 2 H).
**(13) 5-[5,6-Bis(methyloxy)-1*H*-benzimidazol-1-yl]-3-{[(3-bromo-4-pyridinyl)methyl]oxy}-2-thiophenecarbonitrile** 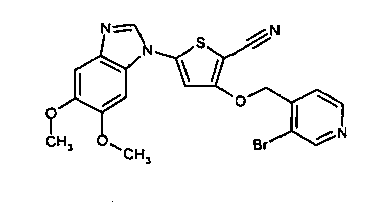 Prepared in a manner analogous to procedure *(1)* using 5-[5,6-bis(methyloxy)-1*H-*benzimidazol-1-yl]-3-{[(3-bromo-4-pyridinyl)-methyl]oxy}-2-thiophenecarboxamide (24.4 mg, 0.05 mol) and the title compound afforded by the trituration procedure described in *(7)* as an off-white solid (7.6 mg). MS [M+1]⁺ 471.19 + 473.19 (characteristic Br isotope pattern); ¹H NMR δ_{H} (400.13 MHz, *d*₆-DMSO, TMS): 8.81 (s, 1 H), 8.66 (d, *J* = 5.0 Hz, 1 H), 8.49 (s, 1 H), 7.76 (s, 1 H), 7.61 (d, *J* = 4.8 Hz, 1 H), 7.35 (s, 1 H), 7.26 (s, 1 H), 5.52, (s, 2 H), 3.84 (s, 3 H), 3.83 (s, 3 H).
**(14) 5-[5,6-Bis(methyloxy)-1*H*-benzimidazol-1-yl]-3-[(cyclohexylmethyl)oxy]-2-thiophenecarbonitrile** 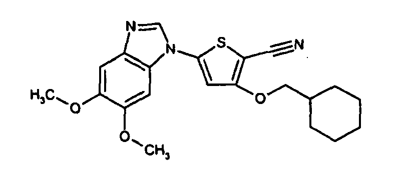 Prepared in manner analogous to procedure *(1)* using 5-[5,6-bis(methyloxy)-1*H-*benzimidazol-1-yl]-3-[(cyclohexylmethyl)oxy]-2-thiophenecarboxamide (20.9 mg, 0.05 mmol) The resulting crude solid was dissolved in 1:1 dimethyl sulfoxide/methanol solution (500 µL) and purified by mass directed auto preparation chromatography to afford the title compound as an off white solid (5.8 mg). MS [M+1]⁺ 398.35; ¹H NMR δ_{H} (400.13 MHz, *d*₆-DMSO, TMS): 8.48 (s, 1 H), 7.63 (s, 1 H), 7.35 (s, 1 H), 7.28 (s, 1 H), 4.14 (d, *J* = 6.0 Hz, 2H), 3.86 (s, 3H), 3.83 (s, 3H), 1.82-1.61 (br. c m, 6H), 1.32-1.01(br. c m, 5H)
**(15) 1-[5-cyano-4-({[2-(trifluoromethyl)phenyl]methyl}oxy)-2-thienyl]-*N*-[2-(4-morpholinyl)ethyl]-1*H*-benzimidazole-5-carboxamide** 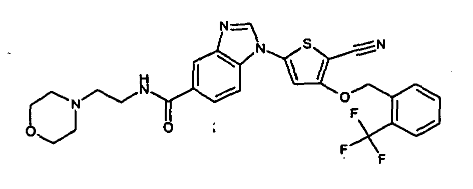 Prepared in an analogous manner to procedure *(1)* using 1-[5-(aminocarbonyl)-4-({[2-(trifluoromethyl)phenyl]methyl)oxy)-2-thienyl]-*N*-[2-(4-morpholinyl)ethyl]-1*H-*benzimidazole-5-carboxamide (28.7 mg, 0.05 mmol). The resulting crude solid was dissolved in 1:1 dimethyl sulfoxide/methanol solution (500 µL) and purified by mass directed auto preparation chromatography to afford the title compound as an off white solid (4.4 mg). MS [M+1]⁺ 556.36; ¹H NMR δ_{H} (400.13 MHz, *d*₆-DMSO, TMS): 8.80 (s, 1 H), 8.55 (t, *J* = 5.5 Hz, 1 H), 8.33 (s, 1 H), 7.97, (m, 1 H), 7.90-7.77 (c m, 5H), 7.67 (t, *J* = 7.5, 1 H), 5.56 (s, 2 H), 3.58 (t, *J* = 4.5 Hz, 4H), 3.43 (q, 6.8 Hz, 2 H), 2.43 (br. s, 4H); other signal (2 H) too broad to observe.
**(16) 5-[5,6-Bis(methyloxy)-1*H*-benzimidazol-1-yl]-3-({(1*R*)-1-[2-(trifluoromethyl)phenyl]ethyl}oxy)-2-thiophenecarbonitrile** 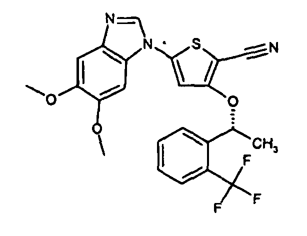 Prepared in an analogous manner to procedure *(1)* using 5-[5,6-bis(methyloxy)-1*H-*benzimidazol-1-yl]-3-({(1*R*)-1-[2-(trifluoromethyl)phenyl]ethyl}oxy)-2-thiophenecarboxamide (24.4 mg, 0.05 mmol). The crude solid was partitioned between dichloromethane (10 mL) and 2M hydrochloric acid solution (5 mL), the organic phase was recovered by passing through a PTFE phase separator and concentrated to dryness by nitrogen blowdown at 40 °C to afford the title compound as an off-white solid (8.2 mg). MS [M+1]⁺ 474.31; ¹H NMR δ_{H} (400.13 MHz, d₆-DMSO, TMS): 8.49 (s, 1H), 7.89-7.75 (c m, 3 H), 7.58 (m, 1 H), 7.41 (s, 1 H), 7.34 (s, 1 H), 7.13 (s, 1 H), 6.04 (q, *J* = 5.8 Hz, 1 H), 3.82 (s, 3 H), 3.81 (s, 3 H), 1.71 (d, *J* = 6.0 Hz, 3 H).
**(17) 5-[5,6-bis(methyloxy)-1*H*-benzimidazol-1-yl]-3-[(2-phenylethyl)oxy]-2-thiophenecarbonitrile** 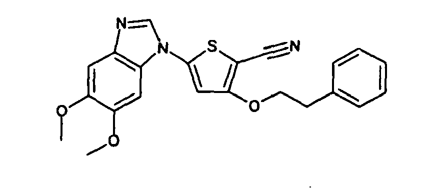 Prepared in an analogous manner to procedure *(1)* using 5-[5,6-bis(methy)oxy)-1*H-*benzimidazol-1-yl]-3-[(2-phenylethyl)oxy]-2-thiophenecarboxamide (21.0 mg, 0.05 mmol). The crude solid was dissolved in 1:1 dimethyl sulfoxide/methanol solution (0.5 mL) and purified by preparative HPLC to afford the title compound as a white solid (5.1 mg). MS [M+1]⁺ 406.14; ¹H NMR δ_{H} (400.13 MHz, *d*₆-DMSO, TMS): 8.47 (s, 1 H), 7.65 (s, 1 H), 7.37-7.23 (cm, 7H), 4.54 (t, *J* = 6.8 Hz, 2 H), 3.85 (s, 3 H), 3.82 (s, 3 H), 3.10 (t, *J* = 6.8 Hz, 2 H).
**(18) 5-[5,6-bis(methyloxy)-1*H*-benzimidazol-1-yl]-3-[({2-[(trifluoromethyl)oxy]phenyl}methyl)oxy]-2-thiophenecarbonitrile** 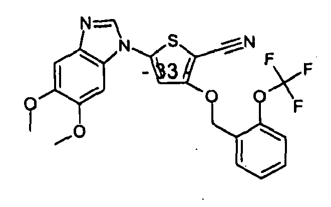 Prepared in an analogous manner to procedure *(1)* using 5-[5,6-bis(methyloxy)-1*H-*benzimidazol-1-yl]-3-[({2-[(trifluoromethyl)-oxy]phenyl}methyl)oxy]-2-thiophenecarbonitrile (24.6 mg, 0.05 mmol). The crude solid was dissolved in 1:1 dimethyl sulfoxide/methanol solution (0.5 mL) and purified by preparative HPLC to afford the title compound as a white solid (3.2 mg). MS [M+1]⁺ 476.09; ¹H NMR δ_{H} (400.13 MHz, d₆-DMSO, TMS): 8.49 (s, 1 H), 7.80 (s, 1 H), 7.75 (dd, *J* = 7.5, 1.3 Hz, 1 H), 7.58 (dd, *J* = 7.8, 1.5 Hz, 1 H), 7.51-7.47 (cm, 2 H), 7.36 (s, 1 H), 7.28 (s, 1 H), 5.48 (s, 2 H), 3.86 (s, 3 H), 3.83 (s, 3 H).
**(19) 5-[5,6-bis(methyloxy)-1*H*-benzimidazol-1-yl]-3-({[2-(methyloxy)phenyl]methyl}oxy)-2-thiophenecarbonitrile** 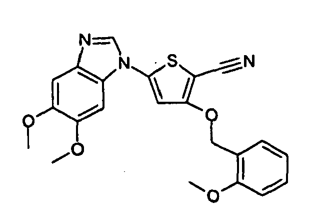 Prepared in an analogous manner to procedure *(1)* using 5-[5,6-bis(methyloxy)-1*H-*benzimidazol-1-yl]-3-({[2-(methyloxy)phenyl]-methyl}oxy)-2-thiophenecarbonitrile (22 mg, 0.05 mmol). The crude solid was dissolved in 1:1 dimethyl sulfoxide/methanol solution (0.5 mL) and purified by preparative HPLC to afford the title compound as a white solid (4.9 mg). MS [M+1]⁺ 422.16; ¹H NMR δ_{H} (400.13 MHz, d₆-DMSO, TMS): 8.50 (s, 1 H), 7.74 (s, 1 H), 7.48 (d, *J* = 7.5 Hz, 1 H), 7.40 (dt, *J* = 1.5, 8.3 Hz, 1 H), 7.63 (s, 1 H), 7.28 (s, 1 H), 7.10 (d, *J* = 8.3, 1 H), 7.01 (t, *J* = 7.3 Hz), 5.39 (s, 2 H), 3.85 (s, 3 H), 3.84 (s, 3 H), 3.83 (s, 3 H).
**(20) 5-[5,6-bis(methyloxy)-1*H*-benzimidazol-1-yl]-3-{[2-(4-morpholinyl)ethyl]oxy}-2-thiophenecarbonitrile** 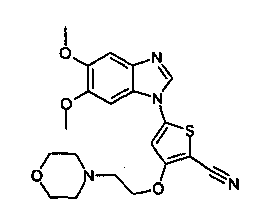 Prepared in an analogous manner to procedure *(1)* using 5-[5,6-bis(methyloxy)-1*H-*benzimidazol-1-yl]-3-{[2-(4-morpholinyl)-ethyl]oxy}-2-thiophenecarboxamide (21.5 mg, 0.05 mmol). The crude solid was subject to the partitioning procedure described in example *(16)* to afford the title compound as a colourless oil (5.2 mg). MS [M+1]⁺ 415.29; ¹H NMR (δ_{H} (400.13 MHz, *d*₆-DMSO, TMS): 8.47 (s, 1 H), 7.65 (s, 1 H), 7.35 (S, 1 H), 7.29 (2, 1 H), 4.44 (t, *J* = 5.5 Hz, 2 H), 3.86 (s, 3 H), 3.83 (s, 3 H), 3.57 (t, *J* = 4.5 Hz, 4H), 2.74 (t, *J* = 5.5 Hz, 2 H), 2.50 (4H multiplet obscured by DMSO signal)
**(21) 5-[5,6-bis(methyloxy)-1*H*-benzimidazol-1-yl]-3-{[2-(2-oxo-1-pyrrolidinyl)ethyl]oxy}-2-thiophenecarbonitrile** 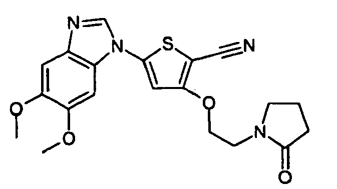 To a solution of 5-[5,6-bis(methyloxy)-1*H*-benzimidazol-1-yl]-3-{[2-(2-oxo-1-pyrrolidinyl)-ethyl]oxy}-2-thiophenecarboxamide (15.2 mg, 0.035 mmol) and pyridine (121 µL, 1.50 mmol) in dichloromethane (5 mL) cooled to approximately -10°C (ice/sodium chloride/water bath) was added trifluoroacetic anhydride (70 µL, 0.5 mmol) and the mixture stirred for 5 mins. after which the solution was allowed to warm up to room temperature over 16 h. The reaction was quenched with 2M aqueous sodium carbonate solution (2 mL) and shaken. The organic layer was recovered by passing the mixture through a PTFE phase separator, collected in a tared glass vial and concentrated to dryness by nitrogen blow down at 40 °C. The resultant crude solid was ultrasonicated in methanol (10 mL) and loaded onto a pre-equilibrated (methanol, 9 mL) aminopropyl functionalised silica cartridge (Isolute, 500 mg) and three column volumes of methanol (9 mL) were allowed to percolate under gravity and collected in tared glass vial. Concentration in a vacuum centrifuge at 50 °C afforded the title compound as an off-white solid (4.9 mg). MS [M+1]⁺ 413.14; ¹H NMR δ_{H} (400.13 MHz, *d*₆-DMSO, TMS): 8.47 (s, 1 H), 7.65 (s, 1 H), 7.35 (s, 1 H), 7.30 (s, 1 H), 4.44 (t, *J* = 5.3 Hz, 2 H), 3.86 (s, 3 H), 3.83 (s, 3 H), 3.59 (t, *J* = 5.3 Hz, 2 H), 3.50 (t, *J* = 7.0 Hz, 2 H), 2.23 (t, *J* = 7.8 Hz, 2 H), 1.94 (quint., *J* = 7.5 Hz (average), 2 H).
**(22) 5-[5,6-bis(methyloxy)-1*H*-benzimidazol-1-yl]-3-[(tetrahydro-2-furanylmethyl)oxy]-2-thiophenecarbonitrile** 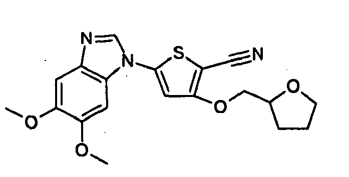 Prepared and isolated in a manner analogous to procedure *(21)* using 5-[5,6-bis(methyloxy)-1*H*-benzimidazol-1-yl]-3-[(tetrahydro-2-furanylmethyl)oxy]-2-thiophenecarboxamide (14.1 mg, 0.035 mmol). The title compound was isolated as an off-white solid (3.3 mg). MS [M+1]⁺ 386.12; ¹H NMR δ_{H} (400.13 MHz, d₆-DMSO, TMS): 8.47 (s, 1 H), 7.63 (s, 1 H), 7.35 (s, 1 H), 7.28 (s, 1 H), 4.39-4.35 (c m, 1 H), 4.30-4.17 (c m, 2 H), 3.86 (s, 3 H), 3.83 (s, 3 H), 3.79 (t, *J* = 6.8 Hz, 1 H), 3.71 (quint., *J* = 7.3 Hz (average), 1 H), 2.07-1.79 (c m, 3 H), 1.72-1.64 (c m, 1 H).
**(23) 5-[5,6-bis(methyloxy)-1*H-*benzimidazol-1-yl]-3-[(tetrahyrdro-2H-pyran-2-ylmethyl)oxy]-2-thiophenecarbonitrile** 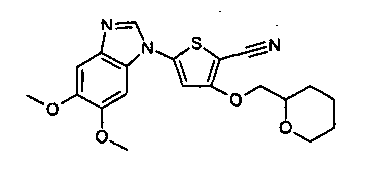 Prepared and isolated in a manner analogous to procedure *(21)* using 5-[5,6-bis(methyloxy)-1*H*-benzimidazol-1-yl]-3-[(tetrahydro-2H-pyran-2-ylmethyl)oxy]-2-thiophene-carboxamide (14.6 mg, 0.035 mmol) The title compound was isolated as an off-white solid (3.3 mg). MS [M+1]⁺400.12; ¹H NMR δ_{H} (400.13 MHz, *d*₆-DMSO, TMS): 8.47 (s, 1 H), 7.62 (s, 1 H), 7.35 (s, 1 H), 7.28 (s, 1 H), 4.29 (d, *J* = 5.0 Hz, 2 H), 3.91 (m, 1 H), 3.86 (s, 3 H), 3.83 (s, 3 H), 3.70-3.66 (cm, 1 H), 3.44-3.37 (cm, 1 H), 1.96-1.81 (cm, 1 H), 1.67-1.43 (cm, 4H), 1.37-1.27 (cm, 1 H).
**(24) 5-[5,6-bis(methyloxy)-1*H*-benzimidazol-1-yl]-3-{[2-(phenyloxy)ethyl]oxy}-2-thiophenecarbonitrile** 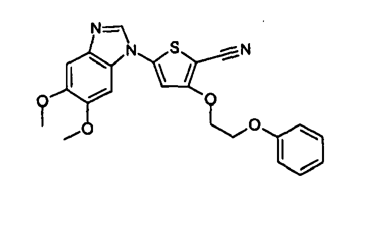 Prepared and isolated in a manner analogous to procedure *(21)* using 5-[5,6-bis(methyloxy)-1*H*-benzimidazol-1-yl]-3-{[2-(phenyl-oxy)ethyl]oxy}-2-thiophenecarboxamide (15.7 mg, 0.035 mmol). The title compound was isolated as an off-white solid (3.3 mg). MS [M+1]⁺ 422.12; ¹H NMR δ_{H} (400.13 MHz, *d*₆-DMSO, TMS): 8.48 (s, 1 H), 7.70 (s, 1 H), 7.35 (s, 1 H), 7.33-7.29 (m, 3 H), 7.00-6.95 (m, 3 H), 4.70 (m, 2 H), 4.37 (m, 2 H), 3.85 (s, 3 H), 3.83 (s, 3 H).
**(25) 5-[5,6-bis(methyloxy)-1*H*-benzimidazol-1-yl]-3-{[(1S)-1-(2-chlorophenyl)butyl]oxy}-2-thiophenecarbonitrile** 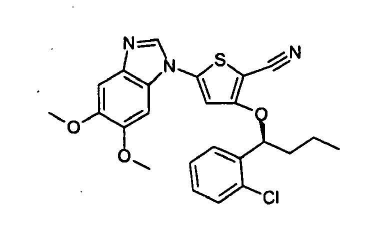 Prepared and isolated in a manner analogous to procedure *(21)* using 5-[5,6-bis(methyloxy)-1*H*-benzimidazol-1-yl]-3-{[(1*S*)-1-(2-chlorophenyl)butyl]oxy}-2-thiophenecarboxamide (17.4 mg, 0.035 mmol). The title compound was isolated as an off-white solid (5.7 mg). MS [M+1]⁺ 468.09 + 470.08 (characteristic chlorine isotope pattern); ¹H NMR δ_{H} (400.13 MHz, d₆-DMSO, TMS): 8.41 (s, 1 H), 7.57 (dd, *J* = 1.0, 7.53 Hz, 1 H), 7.48 (d, *J* = 7.8 Hz, 1 H), 7.44 (t, *J* = 6.8 Hz, 1 H), 7.38-7.32 (cm, 3 H), 7.08 (s, 1 H), 5.90 (m, 1 H), 3.81 (s, 1 H), 3.80 (s, 3 H), 2.07-1.86 (cm, 2 H), 1.56-1.37 (m, 2 H), 0.96 (t, *J* = 7.3 Hz, 3 H).
**(26) 5-[5,6-bis(methyloxy)-1*H*-benzimidazol-1-yl]-3-[(3-thienylmethyl)oxy]-2-thiophenecarbonitrile** 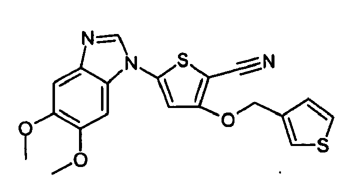 Prepared and isolated in a manner analogous to procedure (21) using 5-[5,6-bis(methyloxy)-1*H*-benzimidazol-1-yl]-3-[(3-thienyl-methyl)oxy]-2-thiophenecarboxamide (14.9 mg, 0.035 mmol). The title compound was isolated as an off-white solid (mg). MS [M+1]⁺ 398.08; ¹H NMR δ_{H} (400.13 MHz, d₆-DMSO, TMS): 8.47 (s, 1 H), 7.74 (overlapping signals, 2 H), 7.62 (dd, *J* = 3.0, 4.8 Hz, 1 H), 7.36 (s, 1 H), 7.24 (overlapping signals, 2 H), 5.42 (s, 2 H), 3.86 (s, 3 H), 3.83 (s, 3 H).
**(27) 5-[5,6-bis(methyloxy)-1*H*-benzimidazol-1-yl]-3-[(2-thienylmethyl)oxyl]-2-thiophenecarbonitrile** 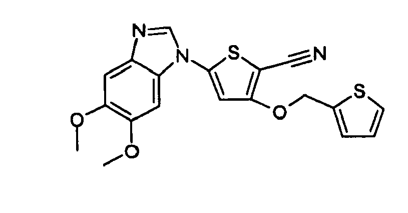 Prepared and isolated in a manner analogous to procedure *(21)* using 5-[5,6-bis(methyloxy)-1*H-*benzimidazol-1-yl]-3-[(2-thienylmethyl)oxy]-2-thiophenecarboxamide (14.5 mg, 0.035 mmol). The title compound was isolated as an clear oil (7.0 mg). MS [M+1]⁺ 398.08; ¹H NMR δ_{H} (400.13 MHz, d₆-DMSO, TMS): 8.47 (s, 1 H), 7.79 (s, 1 H), 7.66 (d, J = 5.3 Hz, 1 H), 7.36 (overlapping signals, 2 H), 7.25 (s, 1 H), 7.08 (dd, *J* = 3.5, 4.8 Hz, 1 H), 5.63 (s, 2 H), 3.87 (s, 3 H), 3.83 (s, 3 H).
**(28) 5-[5,6-bis(methyloxy)-1*H*-benzimidazol-1-yl]-3-{[(1*R*)-1-methylpropyl]oxy}-2-thiophenecarbonitrile** 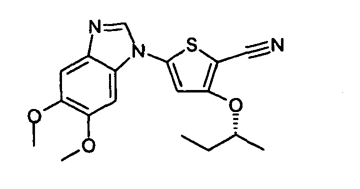 Prepared and isolated in a manner analogous to procedure (21) using 5-[5,6-bis( methyloxy)-1*H*-benzimidazol-1-yl]-3-{[(1*R*)-1-methylpropyl]oxy}-2-thiophenecarboxamide (13.6 mg, 0.035 mmol). The title compound was isolated as an off-white solid (5.3 mg). MS [M+1]⁺ 358.11; ¹H NMR δ_{H} (400.13 MHz, d₆-DMSO, TMS): 8.49 (s, 1 H), 7.64 (s, 1 H), 7.35 (s, 1 H), 7.28 (s, 1 H), 4.66 (sext., *J* = 6.0 Hz, 1 H), 3.86 (s, 3 H), 3.83 (s, 3 H), 1.71 (cm, 2 H), 1.34 (d, *J* = 6.3 Hz, 3 H), 0.96 (t, *J* = 7.3 Hz, 3 H).

### Biological data

### IKK3 assay

Recombinant human IKK3 was expressed in baculovirus as a FLAG tagged fusion protein, and its activity was assessed using a time-resolved fluorescence resonance energy transfer (TR-FRET) assay. Briefly, IKK3 (15 nM final) diluted in assay buffer (50 mM HEPES, 10 mM MgCl₂, 1 mM CHAPS pH 7.4 with 1 mM DTT and 0.01 % w/v BSA) was added to wells containing various concentrations of compound or DMSO vehicle (3% final). The reaction was initiated by the addition of GST-IκBα substrate (25 nM final)/ATP (2 µM final), in a total volume of 30 µl. The reaction was incubated for 15 minutes at room temperature, then terminated by the addition of 15 µl of 50 mM EDTA. Detection reagent (15 µl) in buffer (100 mM HEPES pH 7.4, 150 mM NaCl and 0.1% w/v BSA) containing antiphosphoserine-IκBα-32/36 monoclonal antibody 12C2 (Cell Signalling Technology, Beverly Massachusetts, USA) labelled with W-1024 europium chelate (Wallac OY, Turku, Finland), and an APC-labelled anti-GST antibody (Prozyme, San Leandro, California, USA) was added and the reaction was further incubated for 45 minutes at room temperature. The degree of phosphorylation of GST-IκBα was measured using a Packard Discovery plate reader (Perkin-Elmer Life Sciences, Pangbourne, UK) as a ratio of specific 665 nm energy transfer signal to reference europium 620 nm signal.

The results are shown in the table below:

| Example | IKK3 Activity |
|---|---|
| 16 | * |
| 13 | * |
| 28 | * |
| 22 | * |
| 2 | * |
| 20 | * |
| 10 | * |
| 19 | * |
| 4 | * |
| | |
| 3 | ** |
| 21 | ** |
| 24 | ** |
| 15 | ** |
| 23 | ** |
| 1 | ** |
| | |
| 25 | ** |
| 17 | ** |
| 18 | ** |
| 27 | ** |
| 5 | ** |
| 6 | ** |
| 14 | ** |
| 8 | ** |
| 26 | ** |
| 11 | ** |
| 7 | ** |
| 9 | ** |
| 12 | ** |

| | |
|---|---|
| * = pIC₅₀ of 5.0 - 6.0; ** = pIC₅₀ of >6.0; | |

## Claims

1. A Compound selected from:
| |
|---|
| 5-[5,6-bis(methyloxy)-1H-benzimidazol-1-yl]-3-({[4-(hydroxymethyl)phenyl]methyl}oxy)-2- thiophenecarbonitrile |
| 5-[5,6-bis(methyloxy)- 1*H*-benzimidazol-1-yl]-3-{[(1*R*)-1-(2-chlorophenyl)ethyl]oxy}-2-thiophenecarbonitrile |
| 5-(*1H*-benzimidazol-1-yl)-3-[(phenylmethyl)oxy]-2-thiophenecarbonitrile |
| 5-[5,6-bis(methyloxy)-*1H*-benzimidazol-1-yl]-3-{[(1-methyl-*1H*-1,2,3-benzotriazol-6-yl)methyl]oxy}-2-thiophenecarbonitrile) |
| 5-(6-(methyloxy)-5-{[2-(4-morpholinyl)ethyl]oxy}-*1H*-benzimidazol-1-yl)-3-{([2-(trifluoromethyl)phenyl]methyl}oxy)-2-thiophenecarbonitrile |
| 5-[5,6-bis(methyloxy)-*1H*-benzimidazol-1-yl]-3-{[(2,5-difluorophenyl)methyl]oxy}-2-thiophenecarbonitrile |
| 5-[5,6-bis(methyloxy)-*1H*-benzimidazol-1-yl]-3-({[2-(methylsulfonyl)phenyl]methyl}oxy)-2-thiophenecarbonitrile |
| 5-(5-chloro-*1H*-benzimidazol-1-yl)-3-[(phenylmethyl)oxy]-2-thiophenecarbonitrile |
| 5-[5,6-bis(methyloxy)-*1H*-benzimidazol-1-yl]-3-{[(2,6-difluorophenyl)methyl]oxy}-2-thiophenecarbonitrile |
| 5-[5-[(3-hydroxypropyl)oxy]-6-(methyloxy)-1 H-benzimidazol-1-yl]-3-({[2-(trifluoromethyl)phenyl]methyl}oxy)-2-thiophene-carbonitrile |
| 5-[5,6-bis(methyloxy)-*1H*-benzimidazol-1-yl]-3-{[(3-bromo-4-pyridinyl)methyl]oxy}-2-thiophenecarbonitrile |
| 5-[5,6-bis(methyloxy)-*1H*-benzimidazol-1-yl]-3-[(cyclohexylmethyl)oxy]-2-thiophenecarbonitrile |
| 1-[5-cyano-4-({[2-(trifluoromethyl)phenyl]methyl}oxy)-2-thienyl]-*N*-[2-(4-morpholinyl)ethyl]-*1H*-benzimidazole-5-carboxamide |
| 5-[5,6-bis(methyloxy)- *1H*-benzimidazol-1-yl]-3-({(1*R*)-1-[2-(trifluoromethyl)phenyl]ethyl}oxy)-2-thiophenecarbonitrile |
| 5-[5,6-bis(methyloxy)-*1H*-benzimidazol-1-yl]-3-[(2-phenylethyl)oxy]-2-thiophenecarbonitrile |
| 5-[5,6-bis(methyloxy)- 1*H*-benzimidazol-1-yl]-3-[({2-[(trifluoromethyl)oxy]phenyl}methyl)oxy]-2-thiophenecarbonitrile |
| 5-[5,6-bis(methyloxy)-*1H*-benzimidazol-1-yl]-3-({[2-(methyloxy)phenyl]methyl}oxy)-2-thiophenecarbonitrile |
| 5-[5,6-bis(methyloxy)-*1H*-benzimidazol-1-yl]-3-{[2-(4-morpholinyl)ethyl]oxy}-2-thiophenecarbonitrile |
| 5-[5,6-bis(methyloxy)-*1H*-benzimidazol-1-yl]-3-{[2-(2-oxo-1-pyrrolidinyl)ethyl]oxy}-2-thiophenecarbonitrile |
| 5-[5,6-bis(methyloxy)-*1H*-benzimidazol-1-yl]-3-[(tetrahydro-2-furanylmethyl)oxy]-2-thiophenecarbonitrile |
| 5-[5,6-bis(methyloxy)-*1H*-benzimidazol-1-yl]-3-[(tetrahydro-*2H*-pyran-2-ylmethyl)oxy]-2-thiophenecarbonitrile |
| 5-[5,6-bis(methyloxy)-*1H*-benzimidazol-1-yl]-3-{[2-(phenyloxy)ethyl]oxy}-2-thiophenecarbonitrile |
| 5-[5,6-bis(methyloxy)-*1H*-benzimidazol-1-yl]-3-{[(1*S*)-1-(2-chlorophenyl)butyl]oxy}-2-thiophenecarbonitrile |
| 5-[5,6-bis(methyloxy)-*1H*-benzimidazol-1-yl]-3-[(3-thienylmethyl)oxy]-2-thiophenecarbonitrile |
| 5-[5,6-bis(methyloxy)-*1H*-benzimidazol-1-yl]-3-[(2-thienylmethyl)oxy]-2-thiophenecarbonitrile |
| 5-[5,6-bis(methyloxy)-*1H*-benzimidazol-1-yl]-3-{[(1*R*)-1-methylpropyl]oxy}-2-thiophenecarbonitrile |
and pharmaceutically acceptable salts or solvates thereof.

2. A pharmaceutical composition, comprising: a therapeutically effective amount of a compound as claimed in claim 1 or a pharmaceutically acceptable salt or solvate thereof and one or more of pharmaceutically acceptable carriers, diluents and excipients.

3. A compound as claimed in claim 1 or a pharmaceutically acceptable salt or solvate thereof for use in therapy.

4. Use of a compound according to claim 1 or a pharmaceutically acceptable salt or solvate for the manufacture of a medicament for the treatment of inflammatory bowel disease, asthma, COPD (chronic obstructive pulmonary disease); osteoarthritis, osteoporosis, fibrotic diseases; dermatosis, including psoriasis, atopic dermatitis and ultraviolet radiation (UV)-induced skin damage; autoimmune diseases including systemic lupus eythematosus, multiple sclerosis, psoriatic arthritis, alkylosing spondylitis, tissue and organ rejection, Alzheimer's disease, stroke, atherosclerosis, restonosis, diabetes, glomerulonephritis, cancer, including Hodgkins disease, cachexia, inflammation associated with infection and certain viral infections, including acquired immune deficiency syndrome (AIDS), adult respiratory distress syndrome, and Ataxia Telangiestasia.

5. A compound as claimed in claim 1 or a pharmaceutically acceptable salt or solvate thereof for the treatment of inflammatory bowel disease, asthma, COPD (chronic obstructive pulmonary disease); osteoarthritis, osteoporosis, fibrotic diseases; dermatosis, including psoriasis, atopic dermatitis and ultraviolet radiation (UV)-induced skin damage; autoimmune diseases including systemic lupus eythematosus, multiple sclerosis, psoriatic arthritis, alkylosing spondylitis, tissue and organ rejection, Alzheimer's disease, stroke, atherosclerosis, restonosis, diabetes, glomerulonephritis, cancer, including Hodgkins disease, cachexia, inflammation associated with infection and certain viral infections, including acquired immune deficiency syndrome (AIDS), adult respiratory distress syndrome, and Ataxia Telangiestasia.

## Patentansprüche

1. Verbindung ausgewählt aus:
5-[5,6-Bis(methyloxy)-1H-benzimidazol-1-yl]-3-({[4-(hydroxymethyl)phenyl]methyl}oxy)-2-thiophencarbonitril,
5-[5,6-Bis(methyloxy)-1H-benzimidazol-1-yl]-3-{[(1R)-(2-chlorphenyl)ethyl]oxy}-2-thiophencarbonitril,
5-(1H-Benzimidazol-1-yl)-3-[(phenylmethyl)oxy]-2-thiophencarbonitril,
5-[5,6-Bis(methyloxy)-1H-benzimidazol-1-yl]-3-{[(1-methyl-1H-1,2,3-benzotriazol-6-yl)methyl]oxy}-2-thiophencarbonitril,
5-(6-(Methyloxy)-5-{[2-(4-morpholinyl)ethyl]oxy}-1H-benzimidazol-1-yl)-3-({[2-(trifluormethyl)phenyl]methyl}oxy)-2-thiophencarbonitril,
5-[5,6-Bis(methyloxy)-1H-benzimidazol-1-yl]-3-{[(2,5-difluorphenyl)methyl]oxy}-2-thiophencarbonitril,
5-[5,6-Bis(methyloxy)-1H-benzimidazol-1-yl]-3-({[2-(methylsulfonyl)phenyl]methyl}oxy)-2-thiophencarbonitril,
5-(5-Chlor-1H-benzimidazol-1-yl)-3-[(phenylmethyl)oxy]-2-thiophencarbonitril,
5-[5,6-Bis(methyloxy)-1H-benzimidazol-1-yl]-3-{[(2,6-difluorphenyl)methyl]oxy}-2-thiophencarbonitril,
5-[5-[(3-Hydroxypropyl)oxy]-6-(methyloxy)-1H-benzimidazol-1-yl]-3-({[2-(trifluormethyl)phenyl]methyl}oxy)-2-thiophencarbonitril,
5-[5,6-Bis(methyloxy)-1H-benzimidazol-1-yl]-3-{[(3-brom-4-pyridinyl)methyl]oxy}-2-thiophencarbonitril, 5-[5,6-Bis(methyloxy)-1H-benzimidazol-1-yl]-3-[cyclohexylmethyl)oxy]-2-thiophencarbonitril,
1-[5-Cyano-4-({[2-(trifluormethyl)phenyl]methyl}oxy)-2-thienyl]-N-[2-(4-morpholinyl)ethyl]-1H-benzimidazol-5-carboxamid,
5-[5,6-Bis(methyloxy)-1H-benzimidazol-1-yl]-3-({(1R)-1-[2-(trifluormethyl)phenyl]ethyl}oxy)-2-thiophencarbonitril,
5-[5,6-Bis(methyloxy)-1H-benzimidazol-1-yl]-3-[(2-phenylethyl)oxy]-2-thiophencarbonitril,
5-[5,6-Bis(methyloxy)-1H-benzimidazol-1-yl]-3-[({2-[(trifluormethyl)oxy]phenyl}methyl)oxy]-2-thiophencarbonitril,
5-[5,6-Bis(methyloxy)-1H-benzimidazol-1-yl]3-({[2-(methyloxy)phenyl]methyl}oxy]-2-thiophencarbonitril, 5-[5,6-Bis(methyloxy)-1H-benzimidazol-1-yl]-3-{[2-(4-morpholinyl)ethyl]oxy}-2-thiophencarbonitril,
5-[5,6-Bis(methyloxy)-1H-benzimidazol-1-yl]-3-{[2-(2-oxo-1-pyrrolidinyl)ethyl]oxy}-2-thiophencarbonitril, 5-[5,6-Bis(methyloxy)-1H-benzimidazol-1-yl]-3-[(tetrahydro-2-furanylmethyl)oxy]-2-thiophencarbonitril,
5-[5,6-Bis(methyloxy)-1H-benzimidazol-1-yl]-3-[(tetrahydro-2H-pyran-2-ylmethyl)oxy]-2-thiophencarbonitril,
5-[5,6-Bis(methyloxy)-1H-benzimidazol-1-yl]-3-{[2-(phenyloxy)ethyl]oxy}-2-thiophencarbonitril,
5-[5,6-Bis(methyloxy)-1H-benzimidazol-1-yl]-3-{[(1S)-1-(2-chlorphenyl)butyl]oxy}-2-thiophencarbonitril,
5-[5,6-Bis(methyloxy)-1H-benzimidazol-1-yl]-3-[(3-thienylmethyl)oxy]-2-thiophencarbonitril,
5-[5,6-Bis(methyloxy)-1H-benzimidazol-1-yl]-3-[(2-thienylmethyl)oxy]-2-thiophencarbonitril,
5-[5,6-Bis(methyloxy)-1H-benzimidazol-1-yl]-3-{[(1R)-1-methylpropyl]oxy}-2-thiophencarbonitril,
und pharmazeutisch annehmbare Salze oder Solvate davon.

2. Pharmazeutische Zusammensetzung, umfassend eine therapeutisch wirksame Menge einer Verbindung gemäß Anspruch 1 oder eines pharmazeutisch annehmbaren Salzes oder Solvats davon und einen oder mehrere von pharmazeutisch annehmbaren Trägern, Verdünnungsstoffen und Exzipienten.

3. Verbindung gemäß Anspruch 1 oder ein pharmazeutisch annehmbares Salz oder Solvat davon zur Verwendung in der Therapie.

4. Verwendung einer Verbindung gemäß Anspruch 1 oder eines pharmazeutisch annehmbaren Salzes oder Solvats zur Herstellung eines Medikaments für die Behandlung von entzündlicher Darmerkrankung, Asthma, COPD (chronisch-obstruktiver Lungenerkrankung); Osteoarthritis, Osteoporose, fibrotischen Erkrankungen; Dermatose, einschließlich Psoriasis, atopischer Dermatitis und durch ultraviolette Strahlung (UV)-induzierter Hautschädigung; Autoimmunerkrankungen, einschließlich systemischer Lupus erythematodes; Multiple Sklerose, psoriatischer Arthritis, Spondylitis ankylosans, Gewebe-und Organabstoßung; Alzheimer-Erkrankung, Schlaganfall, Atherosklerose, Restenose, Diabetes, Glomerulonephritis, Krebs, einschließlich Hodgkins-Erkrankung, Kachexie, Entzündung, die mit Infektion und bestimmten viralen Infektionen assoziiert ist, einschließlich erworbenes Immundefizienzsyndrom (AIDS), Atemwegsblockade des Erwachsenen und Ataxia Teleangiectasia.

5. Verbindung gemäß Anspruch 1 oder ein pharmazeutisch annehmbares Salz oder Solvat davon für die Behandlung von entzündlicher Darmerkrankung, Asthma, COPD (chronisch-obstruktiver Lungenerkrankung);
Osteoarthritis, Osteoporose, fibrotischen Erkrankungen; Dermatose, einschließlich Psoriasis, atopischer Dermatitis und durch ultraviolette Strahlung (UV)-induzierter Hautschädigung; Autoimmunerkrankungen, einschließlich systemischer Lupus erythematodes; Multiple Sklerose, psoriatischer Arthritis, Spondylitis ankylosans, Gewebe- und Organabstoßung; Alzheimer-Erkrankung, Schlaganfall, Atherosklerose, Restenose, Diabetes, Glomerulonephritis, Krebs, einschließlich Hodgkins-Erkrankung, Kachexie, Entzündung, die mit Infektion und bestimmten viralen Infektionen assoziiert ist, einschließlich erworbenes Immundefizienzsyndrom (AIDS), Atemwegsblockade des Erwachsenen und Ataxia Teleangiectasia.

## Revendications

1. Composé choisi parmi :
| |
|---|
| 5-[5,6-bis(méthyloxy)-1*H*-benzimidazol-1-yl]-3-({[4-(hydroxy-méthyl)phényl]méthyl}oxy)-2-thiophènecarbonitrile |
| 5-[5,6-bis(méthyloxy)-1*H*-benzimidazol-1-yl]-3-{[(1*R*)-1-(2-chlorophényl)éthyl]oxy}-2-thiophènecarbonitrile |
| 5-(1*H*-benzimidazol-1-yl)-3-[(phénylméthyl)oxy]-2-thiophène-carbonitrile |
| 5-[5,6-bis(méthyloxy)-1*H*-benzimidazol-1-yl]-3-{[(1-méthyl-1*H*-1,2,3-benzotriazol-6-yl)méthyl]oxy}-2-thiophènecarbonitrile |
| 5-(6-(méthyloxy)-5-{[2-(4-morpholinyl)éthyl]oxy}-1*H*-benzimidazol-1-yl)-3-({[2-(trifluorométhyl)phényl]méthyl}oxy)-2-thiophènecarbonitrile |
| 5-[5,6-bis(méthyloxy)-1*H*-benzimidazol-1-yl]-3-{[(2,5-difluorophényl)méthylloxyl-2-thiophènecarbonitrile |
| 5-[5,6-bis(méthyloxy)-1*H*-benzimidazol-1-yl]-3-({[2-(méthyl-sulfonyl)phényl]méthyl}oxy)-2-thiophènecarbonitrile |
| 5-(5-chloro-1*H*-benzimidazol-1-yl)-3-[(phénylméthyl)oxy]-2-thiophènecarbonitrile |
| 5-[5,6-bis(méthyloxy)-1*H*-benzimidazol-1-yl]-3-{[(2,6-difluoro-phényl)méthyl]oxy}-2-thiophènecarbonitrile |
| 5-[5-[(3-hydroxypropyl)oxy]-6-(méthyloxy)-1*H*-benzimidazol-1-yl]-3-({[2-(trifluorométhyl)phényl]méthyl}oxy)-2-thiophène-carbonitrile |
| 5-[5,6-bis(méthyloxy)-1*H*-benzimidazol-1-yl]-3-{[(3-bromo-4-pyridinyl)méthyl]oxy}-2-thiophènecarbonitrile |
| 5-[5,6-bis(méthyloxy)-1*H*-benzimidazol-1-yl]-3-[(cyclohexyl-méthyl)oxy]-2-thiophènecarbonitrile |
| 1-[5-cyano-4-({[2-(trifluorométhyl)phényl]méthyl}oxy)-2-thiényl]-*N*-[2-(4-morpholinyl)éthyl]-1*H*-benzimidazole-5-carboxamide |
| 5-[5,6-bis(méthyloxy)-1*H*-benzimidazol-1-yl]-3-({[(1*R*)-1-[2-(trifluorométhyl)phényl]éthyl}oxy)-2-thiophènecarbonitrile |
| 5-[5,6-bis(méthyloxy)-1*H*-benzimidazol-1-yl]-3-[(2-phényl-éthyl)oxy]-2-thiophènecarbonitrile |
| 5-[5,6-bis(méthyloxy)-1*H*-benzimidazol-1-yl]-3-[({2-[(trifluoro-méthyl)oxy]phényl}méthyl)oxy]-2-thiophènecarbonitrile |
| 5-[5,6-bis(méthyloxy)-1*H*-benzimidazol-1-yl]-3-({[2-(méthyl-oxy)phényl]méthyl}oxy)-2-thiophènecarbonitrile |
| 5-[5,6-bis(méthyloxy)-1*H*-benzimidazol-1-yl]-3-{[2-(4-morpholinyl)éthyl]oxy}-2-thiophènecarbonitrile |
| 5-[5,6-bis(méthyloxy)-1*H*-benzimidazol-1-yl]-3-{[2-(2-oxo-1-pyrrolidinyl)éthyl]oxy}-2-thiophènecarbonitrile |
| 5-[5,6-bis(méthyloxy)-1*H*-benzimidazol-1-yl]-3-[(tétrahydro-2-furanylméthyl)oxy]-2-thiophènecarbonitrile |
| 5-[5,6-bis(méthyloxy)-1*H*-benzimidazol-1-yl]-3-[(tétrahydro-2*H*-pyran-2-ylméthyl)oxy]-2-thiophènecarbonitrile |
| 5-[5,6-bis(méthyloxy)-1*H*-benzimidazol-1-yl]-3-{[2-(phényloxy)-éthyl]oxy}-2-thiophènecarbonitrile |
| 5-[5,6-bis(méthyloxy)-1*H*-benzimidazol-1-yl]-3-{[(1S)-1-(2-chlorophényl)butyl]oxy}-2-thiophènecarbonitrile |
| 5-[5,6-bis(méthyloxy)-1*H*-benzimidazol-1-yl]-3-[(3-thiényl-méthyl)oxy]-2-thiophènecarbonitrile |
| 5-[5,6-bis(méthyloxy)-1*H*-benzimidazol-1-yl]-3-[(2-thiényl-méthyl)oxy]-2-thiophènecarbonitrile |
| 5-[5,6-bis(méthyloxy)-1*H*-benzimidazol-1-yl]-3-{[(1R)-1-méthyl-propyl]oxy}-2-thiophènecarbonitrile |
et des sels ou des solvates pharmaceutiquement acceptables de celui-ci.

2. Composition pharmaceutique, comprenant : une quantité thérapeutiquement efficace d'un composé selon la revendication 1 ou d'un sel ou d'un solvate pharmaceutiquement acceptable de celui-ci et un ou plusieurs supports, diluants et excipients pharmaceutiquement acceptables.

3. Composé selon la revendication 1 ou un sel ou un solvate pharmaceutiquement acceptable de celui-ci en vue d'une d'utilisation en thérapie.

4. Utilisation d'un composé selon la revendication 1 ou d'un sel ou d'un solvate pharmaceutiquement acceptable pour la fabrication d'un médicament destiné au traitement d'une maladie intestinale inflammatoire, l'asthme, la BPCO (bronchopneumopathie chronique obstructive) ; l'arthrose, l'ostéoporose, de maladies fibrotiques ; d'une dermatose, y compris le psoriasis, la dermite atopique et des lésions cutanées induites par des rayons ultraviolets (UV) ; de maladies auto-immunes y compris le lupus érythémateux systémique, la sclérose en plaques, l'arthrite psoriasique, la spondylarthrite ankylosante, le rejet de tissu et d'organe, la maladie d'Alzheimer, l'accident vasculaire cérébral, l'athérosclérose, la resténose, le diabète, la glomérulonéphrite, le cancer, y compris la maladie de Hodgkin, la cachexie, une inflammation associée à une infection et certaines infections virales, y compris le syndrome de l'immunodéficience acquise (SIDA), le syndrome de détresse respiratoire de l'adulte et l'ataxie télangiectasique.

5. Composé selon la revendication 1 ou un sel ou un solvate pharmaceutiquement acceptable de celui-ci destiné au traitement d'une maladie intestinale inflammatoire, l'asthme, la BPCO (bronchopneumopathie chronique obstructive) ; l'arthrose, l'ostéoporose, de maladies fibrotiques ; d'une dermatose, y compris le psoriasis, la dermite atopique et des lésions cutanées induites par des rayons ultraviolets (UV) ; de maladies auto-immunes y compris le lupus érythémateux systémique, la sclérose en plaques, l'arthrite psoriasique, la spondylarthrite ankylosante, le rejet de tissu et d'organe, la maladie d'Alzheimer, l'accident vasculaire cérébral, l'athérosclérose, la resténose, le diabète, la glomérulonéphrite, le cancer, y compris la maladie de Hodgkin, la cachexie, une inflammation associée à une infection et certaines infections virales, y compris le syndrome de l'immunodéficience acquise (SIDA), le syndrome de détresse respiratoire de l'adulte et l'ataxie télangiectasique.
